(19) Europäisches Patentamt
European Patent Office
Office européen des brevets

(11) Publication number: **0 015 110 B1**

(12) **EUROPEAN PATENT SPECIFICATION**

(45) Date of publication of patent specification: **21.08.85**

(21) Application number: **80300387.0**

(22) Date of filing: **11.02.80**

(51) Int. Cl.$^4$: **A 61 K 45/06,** A 61 K 35/66, C 07 C 127/19 // (A61K35/66, 31:17),(A61K35/66, 31:64)

(54) **Anticoccidial composition, carbanilides and method of preparing them.**

(30) Priority: **14.02.79 US 12165**

(43) Date of publication of application: **03.09.80 Bulletin 80/18**

(45) Publication of the grant of the patent: **21.08.85 Bulletin 85/34**

(84) Designated Contracting States: **BE CH DE FR IT LU NL SE**

(56) References cited:
**DE-A-2 334 355**
**FR-A-1 558 257**
**FR-A-2 125 026**
**FR-A-2 126 355**
**FR-A-2 345 160**
**GB-A-1 339 467**
**GB-A-1 463 519**
**US-A-2 823 162**
**US-A-3 627 883**

(73) Proprietor: **ELI LILLY AND COMPANY**
**307, East McCarty Street**
**Indianapolis Indiana 46285 (US)**

(72) Inventor: **Callender, Maurice Emerson**
**1243 North Eaton**
**Indianapolis Indiana (US)**
Inventor: **Jeffers, Thomas Kirk**
**1303 Bittersweet Drive**
**Greenfield Indiana (US)**
Inventor: **O'Doherty, George Oliver Plunkett**
**R.R. 1. Box 260**
**Greenfield Indiana (US)**
Inventor: **Clinton, Albert James**
**3107, Osceola Lane**
**Indianapolis Indiana (US)**

(74) Representative: **Crowther, Terence Roger et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 015 110 B1

**Description**

This invention relates to anticoccidial compositions, such as feedstuffs or premixes, destined for consumption by avian species, particularly poultry such as chickens or turkeys, and which compositions contain as the active ingredients a combination of an antibiotic and a urea derivative.

Coccidiosis has long constituted a problem of major commercial significance in the rearing of poultry. If untreated, the severe forms of the disease lead to poor weight gain, reduced feed efficiency and high mortality. Although many agents have been discovered which are, to a greater or lesser extent, effective against the *Eimeria*—the genera of protozoans principally responsible for coccidiosis—it is unfortunately the case that the *Eimeria* show a propensity to develop drug resistant strains, see *Advances in Pharmacology and Chemotherapy, II*, 221—293 (1973). There is thus a continuing need for the provision of new chemotherapeutic regimes capable of controlling this disease.

Polyether antibiotics are known to be effective in the control of coccidiosis, see for example U.S. Patent No. 3,501,568, as are carbanilide derivatives, see for example U.S. Patents Nos. 2,731,382 and 3,284,433. US—A—3627883 discloses mixtures of a polyether antibiotic, X206, with a carbanilide, while GB—A—1339467 discloses combinations of two coccidiostats. However, heretofore, it has not been appreciated that a combination of a polyether antibiotic and a carbanilide derivative exhibits surprisingly enhanced activity (i.e. synergism and potentiation) against coccidiosis-producing strains of *Eimeria*.

Accordingly, the present invention provides an anticoccidial composition for consumption by an avian species; characterized in that the composition comprises as active ingredients a polyether antibiotic selected from monensin (factors A, B, and C), laidlomycin, grisorixin, lenoremycin, salinomycin, narasin, lonomycin, alborixin, antibiotic A204, A32887 (K41), etheromycin, lasalocid (factors A, B, C, D, and E), isolasalocid A, lysocellin, mutalomycin, or antibiotic A23187 and a carbanilide derivative, the ratio of carbanilide to polyether being in the range of from 40:1 to 1:20, associated with a carrier or feedstuff.

The polyether antibiotics are a class of antibiotics produced by the *Streptomyces* genus of microorganisms. They comprise a multiplicity of cyclic ethers in their structures. The class is reviewed in *Kirk-Othmer*: *Encyclopedia of Chemical Technology*, Vol. 3, Third Edition (John Wiley & Sons, Ind., 1978), page 47 *et seq.*; in *Annual Reports in Medicinal Chemistry Volume 10* (Academic Press, N. Y. 1975), page 246 *et seq.*; and in *J. Chrom. Lib.*, Vol. 15 (Elsevier Scientific Publishing Co., N. Y. 1978), page 488 *et seq.*

Like other products of fermentation origin, many of the polyether antibiotics comprise more than one factor. The various factors are all usable in the present invention. Further, many of these antibiotics readily form ethers, esters or salts, which are either active as such or are converted *in vivo* to the basic antibiotic. Such derivatives can also be employed in the present invention. All that is necessary is that an active moiety of a polyether antibiotic be delivered *in vivo*.

Preferred polyether antibiotics include mutalomycin, monensin, narasin, lasalocid, salinomycin A204 and lonomycin, and especially monensin, narasin, lasalocid, salinomycin and A-204. Monensin is the presently preferred polyether antibiotic for use in the invention, particularly in the commercially available form, i.e. that form consisting of factor A and a minor amount of factor B.

The term "carbanilide derivative" as used herein refers to that particular class of urea derivatives containing the moiety:

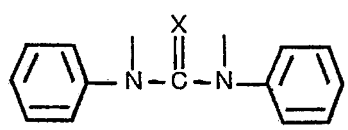

where X is oxygen or sulfur and where each phenyl ring may be substituted. The term also embraces simple complexes of these ureas, for example the complex formed between 4',4-dinitrocarbanilide and 2-hydroxy-4,6-dimethylpyrimidine and known as "nicarbazin".

Preferred carbanilide derivatives are those which possess the structural formula (I):

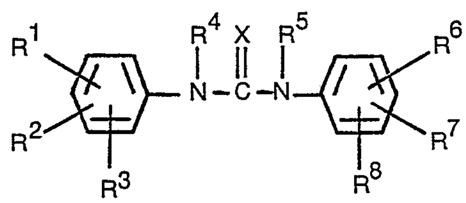

(I)

where $R^1$, $R^2$ and $R^3$ are the same or different and can each represent hydrogen; halogen; cyano; amino; nitro; $C_{1-6}$alkyl; $C_{1-4}$alkoxy; $C_{2-4}$alkanoylamino; $C_{1-4}$alkylthio; $C_{1-4}$haloalkyl; $C_{1-4}$haloalkoxy; $C_{1-4}$haloalkylthio; $C_{1-6}$alkoxycarbonyl; $C_{2-4}$haloalkenyloxy; $C_{1-4}$alkoxycarbonylthio; $C_{1-4}$alkylsulfonyl; $C_{1-4}$haloalkylsulfonyl; fluorosulfonyl; phenoxy optionally substituted by halogen, $C_{1-4}$haloalkyl or nitro; phenoxycarbonyl optionally substituted by $C_{1-4}$alkyl; phenyl; or phenylsulfonyl optionally substituted by nitro, acetamido, isopropylideneamino or a group of the formula

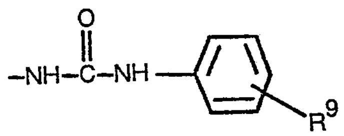

where $R^9$ is $C_{1-4}$haloalkylthio or $C_{2-4}$haloalkenyloxy; $R^4$ and $R^5$ are the same or different and can each represent hydrogen or $C_{1-4}$alkyl; $R^6$, $R^7$ and $R^8$ are the same or different and can each represent hydrogen; halo; cyano; amino; $C_{2-4}$alkanoylamino; N-$C_{1-4}$alkyl $C_{2-4}$alkanoylamino; nitro; $C_{1-6}$alkyl; $C_{1-4}$alkoxy; $C_{1-4}$alkylthio, $C_{1-4}$haloalkyl; $C_{1-4}$haloalkoxy, $C_{1-4}$haloalkylthio, $C_{2-4}$haloalkenyloxy; $C_{1-4}$haloalkylsulfonyl; $C_{1-6}$alkoxycarbonyl, aminosulfonyl or benzoyl; provided that at least one of $R^1$, $R^2$, $R^3$, $R^6$, $R^7$ and $R^8$ is other than hydrogen, and complexes of such compounds with 2-hydroxy-4,6-dimethylpyrimidine.

It is preferred that there be substituents at the *para*-positions of the phenyl rings of the carbanilide moieties, particularly for electronegative substituents, *meta*-substitution is less preferred and *ortho*-substitution least preferred. In general, compounds having amino substitution are not very active and the acyl derivatives of such compounds are preferably utilized.

X is preferably oxygen.

Many of the carbanilides of value in the present invention are known, some also being described as possessing anticoccidial activity.

The following list illustrates specific carbanilides of value in the invention and includes where known and or appropriate, relevant literature references:

Group A

A.1. 3,3'-bis(trifluoromethyl)-4,4'-dichlorocarbanilide (CAS registry #370—50—3; C.A. *68*:2655v)
A.2. 3,3',5,5'-tetrakis(trifluoromethyl)carbanilide (CAS registry #3824—74—6; C.A. *66*: P646444, C.A. *68*: 2655v, and C.A. *71*: P91052y)
A.3. 3-chloro-4'-fluorocarbanilide (C.A. *65*: 12792c)
A.4. 2-chloro-2'-fluorocarbanilide
A.5. 3,4',5-tris(trifluoromethyl)carbanilide (CAS registry #23747—71—9; C.A. *71*: P91056c)
A.6. 3,4,5-trichlorocarbanilide
A.7. 2-chloro-5-(trifluoromethyl)-4'-(ethoxycarbonyl)carbanilide
A.8. 2,6-dimethyl-4'-(N-methylacetamido)carbanilide
A.9. 2-methoxy-4'-acetamidocarbanilide
A.10. 3-(trifluoromethyl)-4'-iodo-thiocarbanilide
A.11. 2-fluoro-4'-(aminosulfonyl)carbanilide
A.12. 2-methoxy-4'-isopropylcarbanilide
A.13. 2-methyl-2',5'-diethoxycarbanilide
A.14. 4-ethyl-2'-methoxycarbanilide
A.15. 2-methyl-5-chloro-2',5'-dimethoxycarbanilide
A.16. 2,4,4'-trimethyl-3'-nitrocarbanilide
A.17. 2-amino-3-nitro-5-(trifluoromethyl)-2',4'-dimethylcarbanilide
A.18. 2-amino-3,4'-dinitro-5-(trifluoromethyl)-2'-chlorocarbanilide
A.19. 2-amino-3,5'-dinitro-5-(trifluoromethyl)-2'-fluorocarbanilide
A.20. 2-amino-3-nitro-5-(trifluoromethyl)-2'-(ethoxycarbonyl)carbanilide
A.21. 2-ethyl-6-sec-butylcarbanilide
A.22. 2-isopropyl-2',4',6'-trimethylcarbanilide
A.23. 2-amino-3-nitro-3',5-bis(trifluoromethyl)-4'-chlorocarbanilide
A.24. 2-ethyl-6-sec-butyl-4'-n-butoxycarbanilide
A.25. 2-amino-3-nitro-5-(trifluoromethyl)-2',4',5'-trichlorocarbanilide
A.26. 2-amino-3,3'-dinitro-5-(trifluoromethyl)-4'-chlorocarbanilide
A.27. 2-amino-3-nitro-5-(trifluoromethyl)-2'-methyl-4'-bromocarbanilide
A.28. 2-amino-3-nitro-5-(trifluoromethyl)-2',6'-dibromo-4'-fluorocarbanilide
A.29. 2-amino-3-nitro-2',5-bis(trifluoromethyl)-4'-chlorocarbanilide
A.30. 3,3',5,5'-tetrakis(trifluoromethyl)thiocarbanilide (CAS registry #1060—92—0; C.A. *66*: 64644H)
A.31. 2,4-dimethoxy-4'-(ethoxycarbonyl)carbanilide
A.32. 4-(ethoxycarbonyl)-2'-methyl-6'-ethylcarbanilide
A.33. 2,2'-dinitro-4,4'-bis(trifluoromethyl)carbanilide (CAS registry #16588—81—1; C.A. *68*: 2655v)
A.34. 3,3',4,4',5,5'-hexachlorocarbanilide
A.35. 3-nitro-4-chloro-4'-(trifluoromethyl)carbanilide
A.36. 4-chloro-3,4'-bis(trifluoromethyl)carbanilide (CAS registry #23747—70—8; C.A. *71*: P91056c)
A.37. 4,4'-dinitro-2,2'-bis(trifluoromethyl)carbanilide (CAS registry #16588—84—4; C.A. *68*: 2655v)
A.38. 4,4'-bis(trifluoromethyl)carbanilide (CAS registry #1960—88—9; C.A. *71*: 91056c)
A.39. 3-bromo-3',5'-dimethylcarbanilide
A.40. 2,5-dichloro-4'-methyl-$N^2$-ethylcarbanilide
A.41. 2,5-dichloro-2',4'-difluorocarbanilide

A.42. 2-amino-3-nitro-3′,5,5′-tris(trifluoromethyl)carbanilide
A.43. 2,6-diethyl-4′-(ethoxycarbonyl)carbanilide
A.44. 3-ethyl-3′-chloro-4′-methyl-$N^2$-ethylcarbanilide
A.45. 2,6-dimethyl-4′-(ethoxycarbonyl)carbanilide
A.46. 4-methoxy-3′-acetamidocarbanilide
A.47. 2-methoxy-4′-(n-butoxycarbonyl)carbanilide
A.48. 4-(isobutoxycarbonyl)carbanilide
A.49. 2,4′-bis(methoxycarbonyl)carbanilide
A.50. 2′,4-dichloro-3-nitro-3′-(trifluoromethyl)carbanilide
A.51. 3,4,4′,5-tetrachloro-3′-(trifluoromethyl)carbanilide (C.A. *63*: P440f)
A.52. 4-chloro-3-nitro-3′,5′-bis(trifluoromethyl)carbanilide
A.53. 2,4,6-trimethyl-4′-(ethoxycarbonyl)carbanilide
A.54. 2-(trifluoromethyl)-2′-ethyl-6′-isopropylcarbanilide
A.55. 4-chloro-3,3′,5′-tris(trifluoromethyl)carbanilide (CAS registry #4528—83—0; C.A. *71*: 91052Y and *66*: 64644h)
A.56. 3,4,4′,5-tetrachloro-3′-nitrocarbanilide
A.57. 2,6-dimethyl-4′-benzoylcarbanilide
A.58. 3,4-dimethyl-2′-ethoxycarbanilide
A.59. 2-chloro-4,4′-bis(methylthio)carbanilide
A.60. 2-methyl-2′-ethoxycarbanilide
A.61. 4-chloro-2-methoxythiocarbanilide
A.62. 4,4′-dinitro-N,N′-dimethylcarbanilide (CAS registry #34594—37—3; C.A. *83*: 36180m and C.A. *85*: 20476t)
A.63. 4-(trifluoromethyl)-4′-nitrocarbanilide (CAS registry #23747—76—4 U.S. 3,867,544)
A.64. 3,3′,5,5′-tetrakis(trifluoromethyl)-N,N′-dimethylcarbanilide
A.65. 4-phenoxy-4′-nitrocarbanilide

A second group of carbanilides of particular value in the present invention are those carbanilides described in U.S. Patent No. 3,284,433. These compounds may be represented by the formula

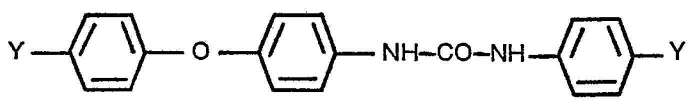

II

wherein each Y is independently selected from the group consisting of chlorine, bromine and nitro radicals; and (2) compounds of the same formula as (II) except that a hydrogen of at least one of the phenyl rings of (II) is replaced by a substituent selected from the group consisting of chlorine, bromine, nitro and $C_{1-6}$alkyl radicals.

Representative compounds of this group include the following:

Group B
B.1. 4-nitro-4′-(4-chlorophenoxy)carbanilide
B.2. 4-nitro-4′-(3,4-dichlorophenoxy)carbanilide
B.3. 4-nitro-3′-chloro-4′-(4-chlorophenoxy)carbanilide
B.4. 4-nitro-3′,5′-dichloro-4′-(4-chlorophenoxy)carbanilide
B.5. 4-nitro-3′-chloro-4′-(3,4-dichlorophenoxy)carbanilide
B.6. 4-nitro-4′-(2,4-dichlorophenoxy)carbanilide
B.7. 4-nitro-4′-(4-nitrophenoxy)carbanilide
B.8. 4-nitro-3′-nitro-4′-(4-chlorophenoxy)carbanilide
B.9. 4-nitro-3′-methyl-4′-(4-chlorophenoxy)carbanilide
B.10. 4-nitro-2′-nitro-4′-(4-chlorophenoxy)carbanilide
B.11. 4-nitro-4′-(2,4-dinitrophenoxy)carbanilide
B.12. 4-nitro-3′-chloro-4′-(2-tert-butyl-4-chlorophenoxy)carbanilide
B.13. 4-nitro-4′-(2-methyl-4-chlorophenoxy)carbanilide
B.14. 4-nitro-3′-bromo-4′-(4-bromophenoxy)carbanilide
B.15. 4-nitro-3′-bromo-4′-(4-chlorophenoxy)carbanilide
B.16. 4-nitro-3′-chloro-4′-(4-bromophenoxy)carbanilide
B.17. 3,3′,4′-trichloro-4-(4-chlorophenoxy)carbanilide
B.18. 3,4′-dichloro-4-(4-chlorophenoxy)carbanilide
B.19. 3,4-dichloro-4′-(4-chlorophenoxy)carbanilide
B.20. 2-methyl-4-nitro-3′-chloro-4′-(4-chlorophenoxy)carbanilide

A third group of carbanilides of value in the present invention are those carbanilides described in German Patent Specification No. 2,334,355. These compounds include the following:

Group C

C.1. 3,3′,5′-tris(trifluoromethyl)-4-methoxycarbanilide
C.2. 3-(trifluoromethoxy)-3′,5′-bis(trifluoromethyl)carbanilide
C.3. 4-(trifluoromethoxy)-3′,5′-bis(trifluoromethyl)carbanilide
C.4. 2,3′,5′-tris(trifluoromethyl)-4-(trifluoromethoxy)carbanilide
C.5. 4-(methylthio)3′,5′-bis(trifluoromethyl)carbanilide
C.6. 4-(methylthio-3′,5′-bis(trifluoromethyl)thiocarbanilide
C.7. 3-chloro-4-(methylthio)-3′,5′-bis(trifluoromethyl)carbanilide
C.8. 3-(trifluoromethylthio)-3′,5′-bis(trifluoromethyl)carbanilide
C.9. 4-(trifluoromethylthio)-3′,5′-bis(trifluoromethyl)carbanilide
C.10. 2-chloro-4-(trifluoromethylthio)-3′,5′-bis(trifluoromethyl)carbanilide
C.11. 4-(trifluoromethylthio)-3′-(trifluoromethyl)-5′-nitrocarbanilide
C.12. 4-(trifluoromethylthio)-2′,5′-bis(trifluoromethyl)-4′-nitrocarbanilide
C.13. 4,4′-bis(trifluoromethylthio)carbanilide
C.14. 4-(trifluoromethylthio)-4′-(chloromethylsulfonyl)carbanilide
C.15. 3-(difluoromethylthio)-3′,5′-bis(trifluoromethyl)carbanilide
C.16. 4-(ethylthio)-3′,5-bis(trifluoromethyl)carbanilide
C.17. 3-chloro-4-(ethylthio)-3′,5′-bis(trifluoromethyl)carbanilide
C.18. 4-ethoxy-3,3′5′-tris(trifluoromethyl)carbanilide
C.19. 3-(trifluoromethyl)-4-ethoxy-4′-(trifluoromethylthio)carbanilide
C.20. 3-(trifluoromethyl)-4-ethoxy-3′-(trifluoromethylthio)carbanilide
C.21. 4-methyl-3-(2-chloroethoxy)-3′,5′-bis(trifluoromethyl)carbanilide
C.22. 4-(1,2-dichlorovinyloxy)-3′-(trifluoromethyl)-4′-chlorocarbanilide
C.23. 4-(1,2-dichlorovinyloxy)-3′,5′-bis(trifluoromethyl)carbanilide
C.24. 4-(2,2,2-trichloro-1,1-difluoroethoxy)-3′,5′-bis(trifluoromethyl)carbanilide
C.25. 2-(2,2-dichloro-1,1-difluoroethoxy)-3′,5′-bis(trifluoromethyl)carbanilide
C.26. 3-(2,2-dichloro-1,1-difluoroethoxy)-3′,5′-bis(trifluoromethyl)carbanilide
C.27. 3-(2,2-dichloro-1,1-difluoroethoxy)-4-bromo-3′,5′-bis(trifluoromethyl)carbanilide
C.28. 4-(2,2-dichloro-1,1-difluoroethoxy)-3′,5′-bis(trifluoromethyl)carbanilide
C.29. 4-(2,2-dichloro-1,1-difluoroethoxy)-3′-(trifluoromethyl)-4′-chlorocarbanilide
C.30. 3-methoxy-4-(2,2-dichloro-1,1-difluoroethoxy)-3′,5′-bis(trifluoromethyl)carbanilide
C.31. 3-methyl-4-(2,2-dichloro-1,1-difluoroethoxy)-3′,5′-bis(trifluoromethyl)carbanilide
C.32. 3-methyl-4-(2,2-dichloro-1,1-difluoroethoxy)-4′-isopropylcarbanilide
C.33. 3-nitro-4-(2,2-dichloro-1,1-difluoroethoxy)-3′,5′-bis(trifluoromethyl)carbanilide
C.34. 2-(2-chloro-1,1,2-trifluoroethoxy)-3′,5′-bis(trifluoromethyl)carbanilide
C.35. 4-(2,2-dichloro-1,1-difluoroethoxy)-3,3′,5′-tris(trifluoromethyl)carbanilide
C.36. 3-(2-chloro-1,1,2-trifluoroethoxy)-3′,5′-bis(trifluoromethyl)carbanilide
C.37. 4-2-chloro-1,1,2-trifluoroethoxy)-3′,5′-bis(trifluoromethyl)carbanilide
C.38. 4-(2-chloro-1,1,2-trifluoroethoxy)-3′,5′-bis(trifluoromethyl)thiocarbanilide
C.39. 4-(2-chloro-1,1,2-trifluoroethoxy)-3′-(trifluoromethyl)-5′-nitrocarbanilide
C.40. 4,4′-bis(2-chloro-1,1,2-trifluoroethoxy)carbanilide
C.41. 4-(2-chloro-1,1,2-trifluoroethoxy)-4′-(trifluoromethylthio)carbanilide
C.42. 4-(2-chloro-1,1,2-trifluoroethoxy)-3′-(trifluoromethyl)-4′-chlorocarbanilide
C.43. 4-(2-chloro-1,1,2-trifluoroethoxy)-3,3′-bis(trifluoromethyl)-5′-nitrocarbanilide
C.44. 4-(2-chloro-1,1,2-trifluoroethoxy)-3,3′,5′-(tristrifluoromethyl)carbanilide
C.45. 3-(1,1,2-trifluoroethoxy)-3′,5′-bis(trifluoromethyl)carbanilide
C.46. 3-(1,1,2,2-tetrafluoroethoxy)-3′,5′-bis(trifluoromethyl)carbanilide
C.47. 3-methyl-4-(1,1,2,2-tetrafluoroethoxy)-3′-(chlorodifluoromethyl)carbanilide
C.48. 3-methyl-4-(1,1,2,2-tetrafluoroethoxy)-3′-(trifluoromethyl)-4′-chlorocarbanilide
C.49. 3-(trifluoromethyl)-4-(1,1,2,2-tetrafluoroethoxy)-4′-bromocarbanilide
C.50. 3-(1,1,2,3,3,3-hexafluoro-n-propoxy)-3′,5′-bis(trifluoromethyl)carbanilide
C.51. 4-(1,1,2,3,3,3-hexafluoro-n-propoxy)-3′,5′-bis(trifluoromethyl)carbanilide
C.52. 3-chloro-4-(1,1,2,3,3,3-hexafluoro-n-propoxy)-3′,5′-bis(trifluoromethyl)carbanilide
C.53. 3-methyl-4-(1,1,2,3,3,3-hexafluoro-n-propoxy)-3′,5′-bis(trifluoromethyl)carbanilide
C.54. 4-(1,1,2,3,3,3-hexafluoro-n-propoxy)-3,3′,5′-tris(trifluoromethyl)carbanilide
C.55. 4-(1,1,2,3,3,3-hexafluoro-n-propoxy)-3,3′-bis(trifluoromethyl)-5′-nitrocarbanilide
C.56. 4-(methoxycarbonylthio)-3′,5′-bis(trifluoromethyl)carbanilide
C.57. 4-(4-chlorophenoxy)-4′-(trifluoromethylthio)thiocarbanilide
C.58. 4-(4-chlorophenoxy)-3′,5′-bis(trifluoromethyl)carbanilide
C.59. 3-chloro-4-(4-chlorophenoxy)-3′,5′-bis(trifluoromethyl)carbanilide
C.60. 4-(4-chlorophenoxy)-3′-(trifluoromethyl)-5′-nitrocarbanilide
C.61. 4-(3,5-bis(trifluoromethyl))phenoxy-3′,5′-bis(trifluoromethyl)carbanilide
C.62. 4-(4-methylphenoxycarbonyl)-3′,5′-bis(trifluoromethyl)carbanilide
C.63. 4-(4-methylphenoxycarbonyl)-4′-(trifluoromethylthio)carbanilide
C.64. 3,5-bis(trifluoromethyl)-2′,4′,6′-trichlorocarbanilide

C.65. 3,5-bis(trifluoromethyl)-2',4',5'-trichlorocarbanilide
C.66. 3,3'-bis(trifluoromethyl)-5'-nitrocarbanilide
C.67. 3,3',5-tris(trifluoromethyl)-5'-nitrocarbanilide
C.68. 2',3,5,6'-tetrakis(trifluoromethyl)-4'-nitrocarbanilide
C.69. 3-(chlorodifluoromethyl)-3',5'-bis(trifluoromethyl)carbanilide
C.70. 3-(1,1,2,2-tetrafluoroethyl)-3',5'-bis(trifluoromethyl)carbanilide
C.71. 4-phenyl-3',5'-bis(trifluoromethyl)-carbanilide
C.72. 3-(fluorosulfonyl)-3',5'-bis(trifluoromethyl)carbanilide
C.73. 4-(fluorosulfonyl)-4'-(trifluoromethylthio)carbanilide
C.74. 4-(fluorosulfonyl)-3'-(trifluoromethyl)-5'-nitrocarbanilide
C.75. 4-(chloromethylsulfonyl)-3',5'-bis(trifluoromethyl)carbanilide
C.76. 2-(ethylsulfonyl)-3',5,5'-tris(trifluoromethyl)carbanilide
C.77. 2-(ethylsulfonyl)-3',5-bis(trifluoromethyl)-5'-nitrocarbonalide
C.78. 4-(trifluoromethylsulfonyl)-3',5'-bis(trifluoromethyl)carbanilide
C.79. 4-(trifluoromethylsulfonyl)-3'-(trifluoromethyl)-4'-methoxycarbanilide
C.80. 4-(trifluoromethylsulfonyl)-4'-(trifluoromethylthio)carbanilide
C.81. 4-(4-nitrophenylsulfonyl)-4'-(trifluoromethylthio)carbanilide
C.82. 4-(4-acetamidophenylsulfonyl)-4'-(trifluoromethylthio)carbanilide
C.83. 4-(4-(isopropylideneamino)phenylsulfonyl)-4'-(trifluoromethylthio)carbanilide
C.84. 4,4-sulfonylbis(3'-(trifluoromethylthio)carbanilide)
C.85. 4,4-sulfonylbis(4'-(1,2-dichlorovinyloxy)carbanilide)
C.86. 4,4-sulfonylbis(3'-(1,1,2,2-tetrafluoroethoxy)carbanilide)
C.87. 4,4-sulfonylbis(4'-(2-chloro-1,1,2-trifluoroethoxy)carbanilide)
C.88. 4,4-sulfonylbis(4'-(trifluoromethylthio)carbanilide)

The presently preferred carbanilide derivative for use in the anticoccidial formulations of the invention is Nicarbazin which is previously stated is a complex of 4,4'-dinitrocarbanilide and 2-hydroxy-4,6-dimethyl-pyrimidine. However, it should be noted that it is the carbanilide portion which exhibits the anticoccidial activity (see *Science 122,* 244 (1955)).

Many carbanilides are known; however, there exists a class of novel carbanilide derivatives which the Applicants have found to be of particular value in the invention. These carbanilides have the structural formula:

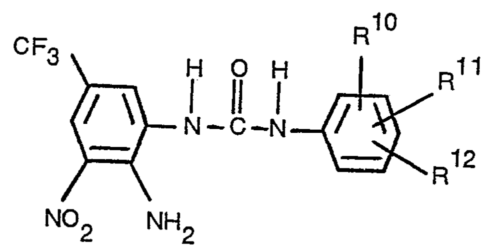

where $R^{10}$, $R^{11}$ and $R^{12}$ are the same or different and can each represent hydrogen, $C_{1-4}$alkyl, nitro, halo, $C_{1-4}$haloalkyl, cyano, or $C_{1-6}$alkoxycarbonyl, provided that at least one of $R^{10}$, $R^{11}$ and $R^{12}$ is other than hydrogen.

Interestingly, although normally free amino substitution is not advantageous in connection with the carbanilides of the invention, despite the presence of the free amino groups, these novel compounds are particularly effective in accordance with the invention.

Examples of this type of compound are:
2-amino-3-nitro-5-(trifluoromethyl)-2',4'-dimethylcarbanilide
2-amino-3,3'-dinitro-5-(trifluoromethyl)-4-'-chlorocarbanilide
2-amino-3-nitro-5-(trifluoromethyl)-2',4',5'-trichlorocarbanilide
2-amino-3-nitro-5-(trifluoromethyl)-2'-(ethoxycarbonyl)carbanilide
2-amino-3,5'-dinitro-5-(trifluoromethyl)-2'-fluorocarbanilide
2-amino-3,4'-dinitro-5-(trifluoromethyl)-2'-chlorocarbanilide
2-amino-3-nitro-3',5-bis(trifluoromethyl)-4'-chlorocarbanilide
2-amino-3-nitro-3',5,5'-tris(trifluoromethyl)carbanilide
2-amino-3-nitro-2',5-bis(trifluoromethyl)-4'-chlorocarbanilide
2-amino-3-nitro-5-trifluoromethyl-2'-methyl-4'-bromocarbanilide and
2-amino-3-nitro-5-(trifluoromethyl-2',6'-dibromo-4'-fluorocarbanilide.

This novel class of ureas, as well as the other carbanilides of the invention, can be prepared by conventional synthetic procedures such as those specified in German Patent Specification No. 2,334,355. For instance, the compounds may be prepared by reaction of the appropriate *o*-phenylenediamine with the corresponding phenyl isocyanate as depicted below:

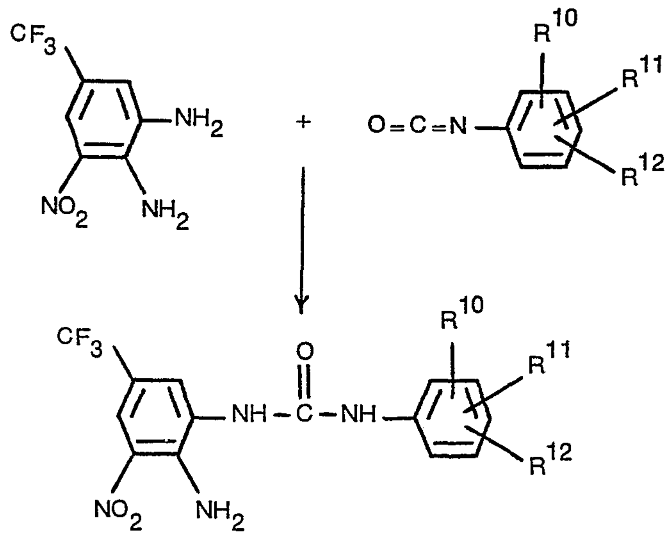

This reaction is preferably carried out at a temperature within the range of 10° to 130°C and in the presence of a tertiary organic base such as pyridine or triethylamine. Any suitable inert organic solvent such as benzene, toluene, chlorobenzene, dioxane or methylene chloride may be utilized.

The following non-limiting Examples illustrate the synthesis of this novel class of carbanilides.

Example 1

2-Amino-3-nitro-5-(trifluoromethyl)-2',4'-dimethylcarbanilide

3-Nitro-5-(trifluoromethyl)-*o*-phenylenediamine (2.2 grams; 0.01 mole) and 2,4-dimethylphenyl isocyanate (1.5 grams; 0.01 mole) were taken up in 40 ml. of methylene chloride, and 1 ml. of pyridine was added. The reaction mixture was maintained at 23°C. for 16 hours. The yellow precipitate was filtered and dried, m.p. >300°C. Elemental analysis showed the following:

| | |
|---|---|
| Calculated for $C_{16}H_{15}F_3N_4O_3$: | C, 52.17; H, 4.08; N, 15.22. |
| Found: | C, 53.01; H, 4.05; N, 16.44. |

Additional carbanilides were prepared by substantially the same procedures as that of Example 1.

Example 2

2-Amino-3,3'-dinitro-5-(trifluoromethyl)-4'-chlorocarbanilide was prepared by reacting 3-nitro-5-(trifluoromethyl)-*o*-phenylenediamine (2.2 grams; 0.01 mole) and 3-nitro-4-chlorophenyl isocyanate (2.0 grams; 0.01 mole). The product, 3.3 grams, melted at 214—216°C. Elemental analysis showed the following:

| | |
|---|---|
| Calculated for $C_{14}H_9ClF_3N_5O_5$: | C, 40.06; H, 2.16; N, 16.96. |
| Found: | C, 40.33; H, 1.88; N, 16.45. |

Example 3

2-Amino-3-nitro-5-(trifluoromethyl)-2',4',5'-trichlorocarbanilide was prepared by reacting 3-nitro-5-(trifluoromethyl)-*o*-phenylenediamine (2.2 grams; 0.1 mole) and 2,4,5-trichlorophenyl isocyanate (2.2 grams; 0.01 mole). The product melted at 225—227°C. Elemental analysis showed the following:

| | |
|---|---|
| Calculated for $C_{14}H_8Cl_3F_3N_4O_3$: | C, 37.91; H, 1.82; N, 12.63. |
| Found: | C, 37.83; H, 1.55; N, 12.40. |

Example 4

2-Amino-3-nitro-5-(trifluoromethyl)-2'-(ethoxycarbonyl)carbanilide was prepared by reacting 3-nitro-5-(trifluoromethyl)-*o*-phenylenediamine (1.1 grams; 0.005 mole) and 2-(ethoxycarbonyl)phenyl isocyanate (1.0 grams; 0.005 mole). The product, 1.4 grams, melted at 186—188°C. Elemental analysis showed the following:

| | |
|---|---|
| Calculated for $C_{17}H_{15}F_3N_4O_5$: | C, 49.52; H, 3.67; N, 13.59. |
| Found: | C, 49.75; H, 3.59; N, 13.50. |

Example 5

2-Amino-3,5′-dinitro-5-(trifluoromethyl)-2′-fluorocarbanilide was prepared from 3-nitro-5-(trifluoromethyl)-*o*-phenylenediamine (1.1 grams; 0.005 mole) and 2-fluoro-5-nitrophenyl isocyanate (0.8 gram; 0.005 mole). The product, 1.2 grams, melted at 218—220°C. Elemental analysis showed the following:

Calculated for $C_{14}H_9F_4N_5O_5$: C, 41.70; H, 2.25; N, 17.37.

Found: C, 41.91; H, 1.98; N, 17.27.

Example 6

2-Amino-3,4′-dinitro-5-(trifluoromethyl)-2′-chlorocarbanilide was prepared by reacting 3-nitro-5-(trifluoromethyl)-*o*-phenylenediamine (1.1 grams; 0.005 mole) and 2-chloro-4-nitrophenyl isocyanate (1.0 gram; 0.005 mole). The product, 1.2 grams, melted at 220—222°C. Elemental analysis showed the following:

Calculated for $C_{14}H_9ClF_3N_5O_5$: C, 40.06; H, 2.16; N, 16.67.

Found: C, 40.11; H, 2.11; N, 16.48.

Example 7

2-Amino-3-nitro-3′,5-bis(trifluoromethyl)-4′-chlorocarbanilide was prepared by reacting 2-amino-3-nitro-5-(trifluoromethyl)-*o*-phenylenediamine (2.2 grams; 0.01 mole) and 4-chloro-3-(trifluoromethyl)phenyl isocyanate (2.2 grams; 0.01 mole). The product, 1.8 grams, melted at 214—216°C. Elemental analysis showed the following:

Calculated for $C_{15}H_9ClF_6N_4O_3$: C, 40.70; H, 2.05; N, 12.66.

Found: C, 40.90; H, 2.04; N, 12.67.

Example 8

2-Amino-3-nitro-3′,5,5′-tris(trifluoromethyl)carbanilide was prepared by reacting 3-nitro-5-(trifluoromethyl)-*o*-phenylenediamine (1.1 grams; 0.005 mole) and (3,5-bis(trifluoromethyl)phenyl)carbamoyl chloride (1.4 grams; 0.005 mole). Elemental analysis of the product showed the following:

Calculated for $C_{16}H_9F_9N_4O_3$: C, 40.35; H, 1.92; N, 11.76.

Found: C, 40.52; H, 1.82; N, 11.78.

Example 9

2-Amino-3-nitro-2′,5-bis(trifluoromethyl)-4′-chlorocarbanilide was prepared by reacting 3-nitro-5-(trifluoromethyl)-*o*-phenylenediamine (2.2 grams; 0.01 mole) and 4-chloro-2-(trifluoromethyl)phenyl isocyanate (2.2 grams; 0.01 mole). The product melted at 245—247°C. Elemental analysis showed the following:

Calculated for $C_{15}H_9ClF_6N_4O_3$: C, 40.70; H, 2.05; N, 12.66.

Found: C, 40.94; H, 1.88; N, 12.55.

Example 10

2-Amino-3-nitro-5-(trifluoromethyl)-2′-methyl-4′-bromocarbanilide was prepared by reacting 3-nitro-5-(trifluoromethyl)-*o*-phenylenediamine (2.2 grams; 0.01 mole) and 2-methyl-4-bromophenyl isocyanate (2.1 grams; 0.01 mole). The product melted at 230—231°C. Elemental analysis showed the following:

Calculated for $C_{15}H_{11}BrF_3N_4O_3$: C, 41.59; H, 2.79; N, 12.93.

Found: C, 41.40; H, 2.65; N, 12.71.

Example 11

2-Amino-3-nitro-5-(trifluoromethyl)-2′,6′-dibromo-4′-fluorocarbanilide was prepared by reacting 3-nitro-5-(trifluoromethyl)-*o*-phenylenediamine (2.2 grams; 0.01 mole) and 2,6-dibromo-4-fluorophenyl isocyanate. The product melted at 249—251°C. Elemental analysis showed the following:

Calculated for $C_{14}H_8Br_2F_4N_4O_3$: C, 32.59; H, 1.56; N, 10.86.

Found: C, 32.64; H, 1.39; N, 10.76.

The ratio of carbanilide to polyether in the anticoccidial compositions of the invention is from 40:1 to 1:20, more preferably from 10:1 to 1:10, for instance 2:1 to 1:2, by weight. Although the compositions of the invention are effective in controlling coccidiosis in all avain species, obviously from the economic viewpoint, the present invention only has commercial significance in coccidiosis treatment in poultry such as chickens or turkeys.

Since coccidiosis affects the intestinal tract, the compositions of the present invention are in a form suitable for oral administration. The polyether antibiotics are generally of low solubility in water, even in the sodium or other salt form. Therefore, the present invention is practiced by administering the polyether/carbanilide combination to the poultry in a feedstuff rather than in drinking water. Furthermore, it is the practice of the industry to supply poultry with only one source of feed, constituting the entire food supply of the poultry. Therefore, in a preferred practice of the present invention, the anticoccidial combinations are supplied in a total feed, with concentrations adjusted accordingly. Those skilled in the art, however, will recognize that concentrations are to be adjusted upward, should it be desired to supply poultry with multiple sources of food only one of which contains the combination of the present invention.

The active ingredients of the compositions of the invention can be employed in a wide range of concentrations in the feed administered to poultry. In general, for those components which are known anticoccidials, the maxima to be employed in accordance with the present invention are the same as the maxima for anticoccidial treatment by the individual components. The lower limits in accordance with the present invention are generally less than for control by the individual components, especially where the components are being used to minimize side effects of either individual component. Generally, compositions in accordance with the invention will not contain less than 15 ppm nor more than 25% by weight of active ingredients.

Representative amounts of selected polyether antibiotics in the final feed destined for consumption are as follows:

from 20 to 120 ppm of monensin;
from 25 to 100 ppm of narasin;
from 35 to 125 ppm of lasalocid.
from 25 to 100 ppm of salinomycin;
from 5 to 15 ppm of A-204;
from 25 to 100 ppm of lonomycin.

The carbanilide is generally employed in a concentration of from 10 to 250 ppm, preferably from 25 to 100 ppm in the poultry feed. Amounts will be adjusted downward where more than one polyether, or more than one carbanilide, is employed. Thus, compositions of the invention in the form of feedstuffs for direct consumption by the avian species to be treated will normally contain from 15 to 450 ppm of the active ingredients.

In a preferred embodiment of the present invention, compositions comprise a polyether antibiotic and nicarbazin or 4,4'-dinitrocarbanilide as the sole anticoccidial agents.

Compositions containing a combination of nicarbazin or 4,4'-dinitrocarbanilide and monensin, especially with the monensin in the commercially available form consisting of factor A and a minor amount of factor B, constitute a particularly preferred embodiment of the invention. A preferred feedstuff for direct feeding of poultry contains from 25 to 75 ppm of monensin and from 25 to 80 ppm of nicarbazin or from 25 to 100 ppm of 4,4'-dinitrocarbanilide.

Since the polyether antibiotics alone are active as anticoccidials, the compositions of the invention are useful regardless of the exact concentration of the carbanilide. As noted above, certain of the carbanilides, alone, exhibit anticoccidial activity. Therefore, preferred embodiments of the present invention are those wherein the carbanilide is employed in a concentration that potentiates the anticoccidial activity of the polyether (where the carbanilide is, itself, lacking anticoccidial activity); or in a concentration that is synergistic with the anticoccidial activity of the polyether (where the carbanilide also exhibits anticoccidial activity).

The Group A compounds include both compounds exhibiting no independent anticoccidial activity, at typical rates, as well as compounds exhibiting independent anticoccidial activity. Each of the Group A compounds has been found to potentiate or synergize, respectively, the activity of a representative polyether antibiotic, monensin. The potentiating and synergizing effect of the Group A compounds is shown in Tables I and IV, below.

The Group B compounds are taught in U.S. Patent No. 3,284,433 to exhibit anticoccidial activity. Therefore, they are preferably employed in amounts which are synergistic as to the anticoccidial activity of the polyethers.

The Group C compounds are taught in German Patent Specification No. 2,334,355 to exhibit anticoccidial activity. They are likewise preferably employed in amounts which are synergistic as to the anticoccidial activity of the polyethers. Data on the effects of the Group C compounds is shown in Tables I, II, and III, below. Simple range-finding experiments as to any of the carbanilides to be employed in the present invention will enable those skilled in the art to determine the preferred potentiating or synergistic amounts of the respective carbanilide.

Poultry feedstuffs of all types and formulae in the poultry industry may be used in administering the combinations of the present invention. The following formulae are exemplary only.

Broiler starter

| Ingredients | Percent |
|---|---|
| Corn, Yellow, Ground | 50.0 |
| Soybean Oil Meal, Solvent Extracted Dehulled (50%) | 30.9 |
| Animal Fat | 6.5 |
| Fish Meal with Solubles (60%) | 5.0 |
| Corn Distillers Dried Solubles | 4.0 |
| Dicalcium Phosphate, Feed Grade | 1.8 |
| Calcium Carbonate (Ground Limestone) | 0.8 |
| Vitamin Premix TK—01 (1.03)1/ | 0.5 |
| Salt (NaCl) | 0.3 |
| Trace Mineral Premix TK—01 (1.02)2/ | 0.1 |
| Methionine Hydroxy Analog | 0.1 |
| Total | 100.0 |

Broiler grower

| Ingredients | Percent |
|---|---|
| Corn, Yellow, Ground | 57.7 |
| Soybean Meal, Solvent, Extracted, Dehulled (50%) | 31.7 |
| Animal Fat (Beef tallow) | 6.0 |
| Dicalcium Phosphate, Feed Grade | 2.7 |
| Calcium Carbonate (Ground Limestone) | 0.9 |
| Vitamin Premix TK—01 (1.03)1/ | 0.5 |
| Salt (NaCl) | 0.2 |
| Methionine Hydroxy Analog | 0.2 |
| Trace Mineral Premix TK—01 (1.02)2/ | 0.1 |
| Total | 100.0 |

Chick starter, light breeds

| Ingredients | | Percent |
|---|---|---|
| Corn, Yellow, Ground | | 56.3 |
| Soybean Meal, Solvent Extracted, Dehulled (50%) | | 17.9 |
| Wheat Middlings | | 10.0 |
| Corn Distillers Dried Solubles | | 5.0 |
| Fish Meal with Solubles | | 5.0 |
| Alfalfa Meal, Dehydrated (17%) | | 2.5 |
| Dicalcium Phosphate, Feed Grade | | 1.3 |
| Calcium Carbonate | | 0.9 |
| Vitamin Premix[1/] | | 0.5 |
| Salt (NaCl) | | 0.3 |
| Methionine Hydroxy Analog | | 0.2 |
| Trace Mineral Premix[2/] | | 0.1 |
| | Total | 100.0 |

Pullet grower

| Ingredients | | Percent |
|---|---|---|
| Corn, Yellow, Ground | | 73.5 |
| Soybean Meal, Solvent Extracted, Dehulled (50%) | | 21.9 |
| Dicalcium Phosphate, Feed Grade | | 2.5 |
| Calcium Carbonate | | 1.0 |
| Vitamin Premix[1/] | | 0.5 |
| Salt (NaCl) | | 0.3 |
| Methionine Hydroxy Analog | | 0.2 |
| Trace Mineral Premix[2/] | | 0.1 |
| | Total | 100.0 |

Pullet developer

| Ingredients | Percent |
|---|---|
| Corn, Yellow, Ground | 67.5 |
| Oats, Ground Whole | 15.0 |
| Soybean Meal, Solvent Extracted, Dehulled (50%) | 13.4 |
| Dicalcium Phosphate, Feed Grade | 2.1 |
| Calcium Carbonate | 1.0 |
| Vitamin Premix[1/] | 0.5 |
| Methionine Hydroxy Analog | 0.3 |
| Salt (NaCl) | 0.2 |
| Trace Mineral Premix[2/] | 0.1 |
| Total | 100.0 |

Turkey starter

| Ingredients | Percent |
|---|---|
| Soybean Meal, Solvent Extracted, Dehulled | 40.7 |
| Corn, Yellow, Ground | 39.7 |
| Fish Meal with Solubles | 5.0 |
| Beef Tallow | 5.0 |
| Corn Distillers Dried Solubles | 2.5 |
| Alfalfa Meal, Dehydrated (17%) | 2.5 |
| Dicalcium Phosphate, Feed Grade | 2.5 |
| Calcium Carbonate | 1.2 |
| Vitamin Premix[1/] | 0.5 |
| Salt (NaCl) | 0.2 |
| Trace Mineral Premix[2/] | 0.1 |
| Methionine Hydroxy Analog | 0.1 |
| Total | 100.0 |

Turkey finisher

| Ingredients | | Percent |
|---|---|---|
| Corn, Yellow, Ground | | 71.2 |
| Soybean Meal, Solvent Extracted, Dehulled (50%) | | 9.9 |
| Corn Distillers Dried Solubles | | 5.0 |
| Alfalfa Meal, Dehydrated (17%) | | 5.0 |
| Animal Fat | | 3.0 |
| Fish Meal with Solubles | | 2.5 |
| Dicalcium Phosphate, Feed Grade | | 1.7 |
| Calcium Carbonate | | 0.5 |
| Vitamin Premix[1] | | 0.5 |
| Salt (NaCl) | | 0.4 |
| Methionine Hydroxy Analog | | 0.2 |
| Trace Mineral Premix[2] | | 0.1 |
| | Total | 100.0 |

1/ Vitamin premix provides 3000 IU of vitamin A, 900 ICU of vitamin D, 40 mg. of vitamin E, 0.7 mg. of vitamin K, 1000 mg. of choline, 70 mg. of niacin, 4 mg. of pantothenic acid, 4 mg. of riboflavin, 0.10 mg. of vitamin $B_{12}$, 0.10 mg. of biotin and 125 mg. of ethoxyquin per kg. of complete feed.

2/ Trace mineral premix provides 75 mg. of manganese, 50 mg. of zinc, 25 mg. of iron and 1 mg. of iodine per kg. of complete feed.

The foregoing compositions are typical of feedstuffs actually administered to poultry. Premixes are commonly used in the poultry industry, and the compositions of the present invention can also take this form. Such premixes typically comprise the polyether antibiotic, the selected carbanilide, and a solid substance capable of being ingested by the poultry, such as corn meal, rice hulls, crushed limestone, soybean meal, soya grits, distillers' dried grains, citrus meal, wheat middlings, clays and the like. Such a premix is mixed or blended with other substances to constitute either an intermediate premix or the finished feed.

A representative premix in accordance with the present invention is as follows:

monensin: 45 grams activity
a selected carbanilide in accordance with the present invention: 45 grams
rice hulls q.s. ad 454 grams

This formulation is mixed thoroughly and can be added to one ton of finished feed to provide a composition in accordance with the invention in the form of a feed suitable for consumption by poultry. Premixes in accordance with the invention will normally contain from 1 to 25% by weight of active ingredients.

The present invention was evaluated in chickens as follows: one-week-old broiler chicks were fed a medicated or control ration, typically for one day, prior to infection (by gavage) with oocysts of a coccidiosis-causing organism, generally 750,000 oocysts of *Eimeria acervulina* and 75,000 oocysts of *Eimeria tenella*. The chicks were maintained on their respective rations for an additional period of time, typically six days. There were five chicks in a group and generally two replicates per treatment. Anticoccidial efficacy was determined by lesion scores. To determine lesion scores, the birds were sacrificed and the severity of lesions scored on a 0—4 scale, with lesion-free birds scored as 0, extremely severe infections scored as 4, and intermediate degrees of infection scored as 1, 2 or 3. The scores of all birds which received a given treatment were averaged.

Most of the compounds were tested in a preliminary test "A"; in this test, monensin was tested alone at 50 ppm and the carbanilide compound was tested in combination with monensin, each at 50 ppm. In a

secondary test "B", monensin and the carbanilide compound were each tested alone, at 50 ppm, and the two were tested in combination, each at 50 ppm. Additional tests at more varied concentrations were also conducted with certain of the compounds.

The results are reported in the following tables. The concentration of monensin and the carbanilides is expressed as the parts per million (ppm) of the total feed provided to the chicks.

TABLE I

| Compound number | | | Lesion scores: *Eimeria acervulina* monensin 0 | 50 | *Eimeria tenella* monensin 0 | 50 |
|---|---|---|---|---|---|---|
| A.3. | A | 0 | 2.8 | 1.6 | 2.0 | 1.9 |
| | | 50 | | 0.7 | | 0.9 |
| | B | 0 | 3.0 | 1.2 | 2.0 | 1.2 |
| | | 50 | 3.0 | 1.2 | 2.5 | 0.7 |
| A.4. | A | 0 | 2.8 | 1.6 | 2.0 | 0.9 |
| | | 50 | | 0 | | 0 |
| | B | 0 | 3.0 | 1.2 | 2.0 | 1.2 |
| | | 50 | 3.0 | 0.9 | 2.4 | 0.6 |
| A.5. | A | 0 | 2.4 | 1.2 | 2.5 | 1.4 |
| | | 50 | | 0.3 | | 0.4 |
| | B | 0 | 3.0 | 1.2 | 2.0 | 1.2 |
| | | 50 | 3.0 | 1.1 | 2.2 | 0 |
| A.6. | A | 0 | 2.8 | 1.6 | 2.0 | 0.9 |
| | | 50 | | 1.6 | | 0.6 |
| | B | 0 | 3.0 | 1.2 | 2.0 | 1.2 |
| | | 50 | 2.9 | 0.8 | 2.5 | 0.5 |
| A.7. | A | 0 | 2.5 | 1.6 | 2.4 | 2.3 |
| | | 50 | | 0.9 | | 0.4 |
| | B | 0 | 3.0 | 1.2 | 2.0 | 1.2 |
| | | 50 | 3.0 | 1.4 | 1.8 | 0.6 |
| A.8. | A | 0 | 3.0 | 1.2 | 2.1 | 0.9 |
| | | 50 | | 0.4 | | 0.2 |
| | B | 0 | 3.3 | 1.3 | 3.0 | 2.2 |
| | | 50 | 2.6 | 0.8 | 3.0 | 1.0 |
| A.9. | A | 0 | 3.0 | 1.2 | 2.1 | 0.9 |
| | | 50 | | 0.7 | | 0.9 |
| | B | 0 | 3.3 | 1.3 | 3.0 | 2.2 |
| | | 50 | 2.8 | 0.8 | 2.9 | 1.3 |
| A.10. | A | 0 | 2.7 | 1.7 | 3.0 | 1.0 |
| | | 50 | | 0.5 | | 0.6 |
| | B | 0 | 3.3 | 1.3 | 3.0 | 2.2 |
| | | 50 | 2.4 | 0.9 | 2.7 | 1.7 |

TABLE I (cont'd)

| Compound number | | | Lesion scores: Eimeria acervulina monensin 0 | Eimeria acervulina monensin 50 | Eimeria tenella monensin 0 | Eimeria tenella monensin 50 |
|---|---|---|---|---|---|---|
| A.11. | A | 0 | 3.6 | 1.2 | 3.0 | 1.2 |
| | | 50 | | 0.6 | | 0.8 |
| | B | 0 | 3.3 | 1.3 | 3.0 | 2.2 |
| | | 50 | 2.6 | 1.4 | 2.8 | 1.1 |
| A.12. | A | 0 | 2.8 | 1.1 | 2.3 | 1.0 |
| | | 50 | | 1.1 | | 0.6 |
| | B | 0 | 3.3 | 1.3 | 3.0 | 2.2 |
| | | 50 | 2.5 | 0.9 | 2.9 | 0.8 |
| A.13. | A | 0 | 3.0 | 1.2 | 2.1 | 0.9 |
| | | 50 | | 0.9 | | 0.5 |
| | B | 0 | 3.3 | 1.3 | 3.0 | 2.2 |
| | | 50 | 2.9 | 1.4 | 3.0 | 1.2 |
| A.14. | A | 0 | 3.0 | 1.2 | 2.1 | 0.9 |
| | | 50 | | 1.2 | | 0.6 |
| | B | 0 | 3.3 | 1.3 | 3.0 | 2.2 |
| | | 50 | 2.6 | 1.1 | 3.0 | 1.0 |
| C.8 | A | 0 | 2.7 | 1.7 | 3.0 | 1.0 |
| | | 50 | | 1.2 | | 0.4 |
| | B | 0 | 3.3 | 1.3 | 3.0 | 2.2 |
| | | 50 | 2.3 | 1.1 | 3.0 | 0.3 |
| A.15. | A | 0 | 3.2 | 1.5 | 2.4 | 1.9 |
| | | 50 | | 1.8 | | 1.0 |
| | B | 0 | 3.3 | 1.3 | 3.0 | 2.2 |
| | | 50 | 2.6 | 1.6 | 2.6 | 1.1 |
| A.16. | A | 0 | 3.0 | 1.1 | 2.6 | 1.0 |
| | | 50 | | 0.9 | | 0.5 |
| | B | 0 | 3.3 | 1.3 | 3.0 | 2.2 |
| | | 50 | 2.8 | 1.0 | 2.8 | 1.1 |
| A.17. | A | 0 | 2.8 | 1.4 | 2.6 | 0.8 |
| | | 50 | | 0.9 | | 0.9 |
| | B | 0 | 2.6 | 1.0 | 2.5 | 1.5 |
| | | 50 | 2.4 | 0.6 | 2.6 | 1.3 |
| A.18. | A | 0 | 2.8 | 1.4 | 2.6 | 0.8 |
| | | 50 | | 0.1 | | 0.3 |
| | B | | 2.6 | 1.0 | 2.5 | 1.5 |
| | | 50 | 2.7 | 0.6 | 2.0 | 0.7 |

TABLE I (cont'd)

| Compound number | | Lesion scores: Eimeria acervulina monensin 0 | 50 | Eimeria tenella monensin 0 | 50 |
|---|---|---|---|---|---|
| A.19. | A 0 | 2.8 | 1.4 | 2.6 | 0.8 |
| | 50 | | 0.4 | | 0.4 |
| | B 0 | 2.6 | 1.0 | 2.5 | 1.5 |
| | 50 | 2.7 | 0.4 | 2.4 | 0.7 |
| A.20. | A 0 | 2.8 | 1.4 | 2.6 | 0.8 |
| | 50 | | 0.5 | | 0.8 |
| | B 0 | 2.6 | 1.0 | 2.5 | 1.5 |
| | 50 | 2.4 | 0.4 | 1.4 | 0.1 |
| A.21. | A 0 | 2.5 | 0.9 | 2.4 | 1.7 |
| | 50 | | 1.3 | | 1.0 |
| | B 0 | 2.6 | 1.0 | 2.5 | 1.5 |
| | 50 | 2.3 | 1.0 | 2.2 | 0.7 |
| A.22. | A 0 | 3.2 | 2.3 | 3.0 | 1.8 |
| | 50 | | 1.3 | | 0.8 |
| | B 0 | 2.6 | 1.0 | 2.5 | 1.5 |
| | 50 | 2.7 | 1.3 | 2.2 | 0.6 |
| A.23. | A 0 | 2.6 | 0.6 | 2.7 | 0.9 |
| | 50 | | 0.5 | | 0.6 |
| | B 0 | 2.6 | 1.0 | 2.5 | 1.5 |
| | 50 | 2.8 | 0.6 | 2.0 | 0.4 |
| A.24. | A 0 | 2.5 | 0.9 | 2.4 | 1.7 |
| | 50 | | 0.9 | | 0.6 |
| | B 0 | 2.6 | 1.0 | 2.5 | 1.5 |
| | 50 | 2.8 | 0.8 | 2.2 | 0.7 |
| A.25. | A 0 | 2.7 | 0.6 | 2.7 | 0.9 |
| | 50 | | 1.1 | | 0.3 |
| | B 0 | 2.6 | 1.0 | 2.5 | 1.5 |
| | 50 | 2.9 | 0.9 | 1.8 | 0.6 |
| A.26. | A 0 | 2.6 | 0.6 | 2.7 | 0.9 |
| | 50 | | 0.7 | | 0.4 |
| | B 0 | 2.6 | 1.0 | 2.5 | 1.5 |
| | 50 | 2.8 | 1.3 | 2.3 | 0.8 |
| A.27. | A 0 | 2.6 | 0.6 | 2.7 | 0.9 |
| | 50 | | 0.3 | | 0.7 |
| | B 0 | 2.6 | 1.2 | 2.6 | 1.5 |
| | 50 | 2.8 | 0.9 | 2.6 | 1.2 |

TABLE I (cont'd)

| Compound number | | Lesion scores: *Eimeria acervulina* monensin 0 | *Eimeria acervulina* monensin 50 | *Eimeria tenella* monensin 0 | *Eimeria tenella* monensin 50 |
|---|---|---|---|---|---|
| A.28. | A 0 | 2.6 | 0.6 | 2.7 | 0.9 |
| | 50 | | 0.4 | | 0.9 |
| | B 0 | 2.6 | 1.2 | 2.6 | 1.5 |
| | 50 | 2.7 | 0.8 | 2.2 | 0.7 |
| A.29. | A 0 | 2.6 | 0.6 | 2.7 | 0.9 |
| | 50 | | 0.8 | | 0.4 |
| | B 0 | 2.6 | 1.2 | 2.6 | 1.5 |
| | 50 | 1.7 | 1.4 | 1.2 | 0.6 |
| A.30. | A 0 | 2.5 | 0.9 | 2.4 | 1.7 |
| | 50 | | 0.8 | | 0.4 |
| | B 0 | 2.6 | 1.2 | 2.6 | 1.5 |
| | 50 | 2.9 | 0.2 | 2.0 | 0.2 |
| A.31. | A 0 | 2.7 | 1.6 | 2.5 | 1.6 |
| | 50 | | 0.3 | | 0.7 |
| | B 0 | 2.6 | 1.2 | 2.6 | 1.5 |
| | 50 | 2.8 | 0.8 | 2.7 | 1.2 |
| | B 0 | 2.4 | 1.2 | 2.2 | 1.8 |
| | 50 | 2.7 | 0.9 | 3.0 | 1.6 |
| A.32. | A 0 | 2.7 | 1.6 | 2.5 | 1.6 |
| | 50 | | 0.4 | | 0.6 |
| | B 0 | 2.6 | 1.2 | 2.6 | 1.5 |
| | 50 | 2.8 | 0.7 | 2.1 | 0.9 |
| | B 0 | 2.4 | 1.2 | 2.2 | 1.8 |
| | 50 | 2.5 | 1.0 | 2.9 | 1.0 |
| A.33. | B 0 | 2.8 | 1.2 | 2.7 | 1.6 |
| | 50 | 2.8 | 0.5 | 2.6 | 1.0 |
| A.34. | B 0 | 2.8 | 1.2 | 2.7 | 1.6 |
| | 50 | 2.9 | 0.3 | 2.2 | 0.3 |
| A.35. | B 0 | 2.8 | 1.2 | 2.7 | 1.6 |
| | 50 | 2.8 | 0.2 | 3.0 | 0.9 |
| A.36. | B 0 | 2.8 | 1.2 | 2.7 | 1.6 |
| | 50 | 2.2 | 0 | 2.6 | 0.1 |
| A.37. | B 0 | 2.8 | 1.2 | 2.7 | 1.6 |
| | 50 | 2.0 | 0.4 | 2.9 | 1.2 |
| A.38. | B 0 | 2.8 | 1.2 | 2.7 | 1.6 |
| | 50 | 2.6 | 0.4 | 2.9 | 1.3 |

TABLE I (cont'd)

| Compound Number | | | Lesion scores: *Eimeria acervulina* monensin 0 | *Eimeria acervulina* monensin 50 | *Eimeria tenella* monensin 0 | *Eimeria tenella* monensin 50 |
|---|---|---|---|---|---|---|
| A.39. | A | 0 | 3.6 | 1.6 | 2.8 | 1.2 |
| | | 50 | | 1.4 | | 0.5 |
| | B | 0 | 3.6 | 2.0 | 2.9 | 1.3 |
| | | 50 | 3.1 | 0.5 | 3.0 | 0.8 |
| A.40. | A | 0 | 3.6 | 1.6 | 2.8 | 1.2 |
| | | 50 | | 1.3 | | 0.3 |
| | B | 0 | 3.6 | 2.0 | 2.9 | 1.3 |
| | | 50 | 2.6 | 0.8 | 2.6 | 0.8 |
| A.41. | A | 0 | 3.6 | 1.6 | 2.8 | 1.2 |
| | | 50 | | 1.3 | | 0.2 |
| | B | 0 | 3.6 | 2.0 | 2.9 | 1.3 |
| | | 50 | 2.1 | 0 | 2.2 | 0.4 |
| A.42. | A | 0 | 2.7 | 1.6 | 2.6 | 1.6 |
| | | 50 | | 0.3 | | 0.7 |
| | B | 0 | 3.6 | 2.0 | 2.9 | 1.3 |
| | | 50 | 3.2 | 2.5 | 2.1 | 0 |
| A.43. | A | 0 | 2.7 | 1.6 | 2.6 | 1.6 |
| | | 50 | | 1.3 | | 0.4 |
| | B | 0 | 3.2 | 1.1 | 1.9 | 0.8 |
| | | 50 | 3.1 | 0.8 | 2.3 | 0.4 |
| A.44. | A | 0 | 3.6 | 1.6 | 2.8 | 1.2 |
| | | 50 | | 1.2 | | 0.4 |
| | B | 0 | 3.2 | 1.1 | 1.9 | 0.8 |
| | | 50 | 2.9 | 0.5 | 2.6 | 0.6 |
| A.45. | A | 0 | 3.0 | 0.9 | 2.8 | 0.5 |
| | | 50 | | 0.8 | | 0.3 |
| | B | 0 | 3.2 | 1.1 | 1.9 | 0.8 |
| | | 50 | 2.7 | 1.0 | 2.1 | 0.2 |
| A.46. | A | 0 | 3.0 | 0.7 | 2.8 | 0.2 |
| | | 50 | | 1.0 | | 0.2 |
| | B | 0 | 3.2 | 1.1 | 1.9 | 0.8 |
| | | 50 | 2.9 | 0.9 | 2.1 | 0.2 |
| A.47. | A | 0 | 2.8 | 1.0 | 3.0 | 1.2 |
| | | 50 | | 1.4 | | 0.7 |
| | B | 0 | 3.2 | 1.1 | 1.9 | 0.8 |
| | | 50 | 2.7 | 0.9 | 2.4 | 0.5 |

TABLE I (cont'd)

| Compound number | | Lesion scores: *Eimeria acervulina* monensin 0 | *Eimeria acervulina* monensin 50 | *Eimeria tenella* monensin 0 | *Eimeria tenella* monensin 50 |
|---|---|---|---|---|---|
| A.48. | A 0 | 2.8 | 1.0 | 3.0 | 1.2 |
| | 50 | | 0.9 | | 0.7 |
| | B 0 | 3.2 | 1.1 | 1.9 | 0.8 |
| | 50 | 3.3 | 1.0 | 2.3 | 0.4 |
| A.49. | A 0 | 3.0 | 0.7 | 2.8 | 0.2 |
| | 50 | | 0.6 | | 0.4 |
| | B 0 | 3.2 | 1.1 | 1.9 | 0.8 |
| | 50 | 3.1 | 0.3 | 2.8 | 0.2 |
| A.50. | B 0 | 2.8 | 1.0 | 1.7 | 0.5 |
| | 50 | 3.0 | 0 | 2.0 | 0 |
| A.51. | B 0 | 2.8 | 1.0 | 1.7 | 0.5 |
| | 50 | 1.3 | 0 | 1.1 | 0 |
| A.52. | B 0 | 2.8 | 1.0 | 1.7 | 0.5 |
| | 50 | 2.8 | 0 | 2.1 | 0.2 |
| A.53. | A 0 | 2.7 | 1.6 | 2.5 | 1.6 |
| | 50 | | 0.8 | | 0.9 |
| | B 0 | 2.4 | 1.2 | 2.2 | 1.8 |
| | 50 | 2.8 | 0.8 | 2.6 | 1.4 |
| A.54. | A 0 | 3.1 | 2.0 | 2.4 | 0.6 |
| | 50 | | 1.7 | | 0.6 |
| | B 0 | 2.4 | 1.2 | 2.2 | 1.8 |
| | 50 | 2.6 | 0.7 | 2.9 | 0.4 |
| A.55. | B 0 | 2.4 | 1.2 | 2.2 | 1.8 |
| | 50 | 2.9 | 0.8 | 2.5 | 0.1 |
| A.56. | B 0 | 2.4 | 1.2 | 2.2 | 1.8 |
| | 50 | 2.8 | 0.6 | 2.8 | 0.2 |
| A.57. | A 0 | 2.7 | 1.6 | 2.5 | 1.6 |
| | 50 | | 0.7 | | 0.9 |
| | B 0 | 2.4 | 1.2 | 2.2 | 1.8 |
| | 50 | 3.0 | 1.2 | 2.8 | 0.9 |
| A.58. | A 0 | 2.7 | 1.6 | 2.5 | 1.6 |
| | 50 | | 0.9 | | 0.7 |
| | B 0 | 2.4 | 1.2 | 2.2 | 1.8 |
| | 50 | 2.7 | 0.7 | 2.7 | 0.8 |
| A.59. | A 0 | 2.9 | 0.9 | 2.2 | 1.7 |
| | 50 | | 0.5 | | 1.4 |
| | B 0 | 2.4 | 1.2 | 2.2 | 1.8 |
| | 50 | 2.9 | 0.7 | 2.9 | 1.1 |

TABLE I (cont'd)

| Compound number | | Lesion scores: *Eimeria acervulina* monensin 0 | *Eimeria acervulina* monensin 50 | *Eimeria tenella* monensin 0 | *Eimeria tenella* monensin 50 |
|---|---|---|---|---|---|
| A.60. | A 0 | 2.7 | 1.6 | 2.5 | 1.6 |
| | 50 | | 0.6 | | 0.7 |
| | B 0 | 2.7 | 1.2 | 2.1 | 2.1 |
| | 50 | 2.9 | 0.8 | 2.7 | 1.6 |
| A.61. | A 0 | 3.0 | 1.5 | 2.8 | 1.9 |
| | 50 | | 0.8 | | 1.7 |
| | B 0 | 2.0 | 0.2 | 2.8 | 1.3 |
| | 50 | 2.0 | 0.7 | 3.1 | 2.2 |
| A.62. | B 0 | 2.0 | 0.2 | 2.8 | 1.3 |
| | 50 | 0.4 | 0 | 2.4 | 0.8 |
| A.63. | B 0 | 2.0 | 0.2 | 2.8 | 1.3 |
| | 50 | 2.6 | 0.1 | 2.9 | 0.6 |
| A.64. | B 0 | 2.0 | 0.2 | 2.8 | 1.3 |
| | 50 | 0.7 | 0 | 2.4 | 0.8 |

TABLE II

| Compound No. | | Lesion scores: *Eimeria acervulina* monensin | | | | | *Eimeria tenella* monensin | | | | |
|---|---|---|---|---|---|---|---|---|---|---|---|
| C.1. | | 0 | 50 | 100 | | | 0 | 50 | 100 | | |
| | 0 | 3.1 | 1.3 | 0.2 | | | 2.0 | 0.5 | 0 | | |
| | 50 | 2.8 | 0.8 | | | | 2.0 | 0.3 | | | |
| | 100 | 2.1 | | | | | 2.1 | | | | |
| | | 0 | 20 | 40 | 80 | 160 | 0 | 20 | 40 | 80 | 160 |
| | 0 | 2.9 | 2.6 | 1.2 | 0.4 | 0 | 2.6 | 2.9 | 2.2 | 0.6 | 0.2 |
| | 40 | 3.0 | | 2.3 | | | 2.7 | | 2.5 | | |
| | 80 | 2.7 | | | 0.2 | | 2.6 | | | 0.5 | |
| | 160 | 2.4 | | | | | 1.9 | | | | |
| C.5. | | 0 | 20 | 40 | 80 | 160 | 0 | 20 | 40 | 80 | 160 |
| | 0 | 2.9 | 2.6 | 1.2 | 0.4 | 0 | 2.6 | 2.9 | 2.2 | 0.6 | 0.2 |
| | 40 | 3.0 | | 1.0 | | | 1.4 | | 1.7 | | |
| | 80 | 2.4 | | | 0.3 | | 2.9 | | | 1.3 | |
| | 160 | 3.0 | | | | | 2.7 | | | | |

TABLE II (cont'd)

Lesion scores

| Compound No. | | Eimeria acervulina monensin | | | | | | Eimeria tenella monensin | | | | | |
|---|---|---|---|---|---|---|---|---|---|---|---|---|---|
| C.6. | | 0 | 50 | 100 | | | | 0 | 50 | 100 | | | |
| | 0 | 3.1 | 1.3 | 0.2 | | | | 2.0 | 0.5 | 0 | | | |
| | 50 | 3.3 | 1.3 | | | | | 2.0 | 0.8 | | | | |
| | 100 | 2.7 | | | | | | 2.4 | | | | | |
| | | 0 | 20 | 40 | 50 | 80 | 160 | 0 | 20 | 40 | 50 | 80 | 160 |
| | 0 | 2.9 | 2.6 | 1.2 | | 0.4 | 0 | 2.6 | 2.9 | 2.2 | | 0.6 | 0.2 |
| | 100 | | | | 1.8 | | | | | | 1.6 | | |
| | 200 | 1.7 | | | | | | 2.0 | | | | | |
| C.8. | | 0 | 50 | 100 | | | | 0 | 50 | 100 | | | |
| | 0 | 3.1 | 1.3 | 0.2 | | | | 2.0 | 0.5 | 0 | | | |
| | 50 | | 0.7 | | | | | | 0.1 | | | | |
| | 100 | | | | | | | | | | | | |
| C.11. | | 0 | 50 | 100 | | | | 0 | 50 | 100 | | | |
| | 0 | 3.1 | 1.3 | 0.2 | | | | 2.0 | 0.5 | 0 | | | |
| | 50 | 2.6 | 0.2 | | | | | 1.9 | 0 | | | | |
| | 100 | 0.2 | | | | | | 0.2 | | | | | |

TABLE II (cont'd)

Lesion scores

| Compound No. | | Eimeria acervulina monensin | | | | | Eimeria tenella monensin | | | | |
|---|---|---|---|---|---|---|---|---|---|---|---|
| C.13. | | 0 | 50 | 100 | | | 0 | 50 | 100 | | |
| | 0 | 3.1 | 1.3 | 0.2 | | | 2.0 | 0.5 | 0 | | |
| | 50 | 0 | 0 | | | | 0.1 | 0 | | | |
| | 100 | 0 | | | | | 0.2 | | | | |
| | | 0 | 20 | 40 | 80 | 160 | 0 | 20 | 40 | 80 | 160 |
| | 0 | 2.9 | 2.6 | 1.2 | 0.4 | 0 | 2.6 | 2.9 | 2.2 | 0.6 | 0.2 |
| | 10 | 2.6 | 1.8 | | | | 3.0 | 1.4 | | | |
| | 20 | 2.4 | 0.7 | | | | 2.8 | 0.7 | | | |
| | 40 | 0.8 | | | | | 1.6 | | | | |
| C.58. | | 0 | 50 | 100 | | | 0 | 50 | 100 | | |
| | 0 | 3.1 | 1.3 | 0.2 | | | 2.0 | 0.5 | 0 | | |
| | 50 | 2.6 | 0.1 | | | | 1.5 | 0 | | | |
| | 100 | 2.8 | | | | | 0.5 | | | | |
| | | 0 | 20 | 40 | 80 | 160 | 0 | 20 | 40 | 80 | 160 |
| | 0 | 2.9 | 2.6 | 1.2 | 0.4 | 0 | 2.6 | 2.9 | 2.2 | 0.6 | 0.2 |
| | 40 | | | 0.7 | | | | | 0.8 | | |
| | 80 | 2.9 | | | | | 2.2 | | | | |
| C.62. | | 0 | 50 | 100 | | | 0 | 50 | 100 | | |
| | 0 | 3.1 | 1.3 | 0.2 | | | 2.0 | 0.5 | 0 | | |
| | 50 | 2.9 | 1.3 | | | | 1.1 | 0.4 | | | |
| | 100 | 2.9 | | | | | 1.9 | | | | |

TABLE II (cont'd)

| Compound No. | | Lesion scores: *Eimeria acervulina* monensin | | | | | Lesion scores: *Eimeria tenella* monensin | | | | |
|---|---|---|---|---|---|---|---|---|---|---|---|
| C.65. | | 0 | 50 | 100 | | | 0 | 50 | 100 | | |
| | 0 | 3.1 | 1.3 | 0.2 | | | 2.0 | 0.5 | 0 | | |
| | 50 | 3.2 | 0.3 | | | | 1.3 | 0 | | | |
| | 100 | 2.8 | | | | | 0.9 | | | | |
| C.76. | | 0 | 50 | 100 | | | 0 | 50 | 100 | | |
| | 0 | 3.1 | 1.3 | 0.2 | | | 2.0 | 0.5 | 0 | | |
| | 50 | 3.0 | 0.8 | | | | 1.9 | 0.2 | | | |
| | 100 | 3.0 | | | | | 1.5 | | | | |
| | | 0 | 20 | 40 | 80 | 160 | 0 | 20 | 40 | 80 | 160 |
| | 0 | 2.9 | 2.6 | 12 | 0.4 | 0 | 2.6 | 2.9 | 2.2 | 0.6 | 0.2 |
| | 40 | 2.1 | | 1.5 | | | 2.6 | | 2.4 | | |
| | 80 | 1.9 | | | 0.2 | | 3.4 | | | 0.9 | |
| | 160 | 2.4 | | | | | 2.7 | | | | |
| C.86. | | 0 | 50 | 100 | | | 0 | 50 | 100 | | |
| | 0 | 3.1 | 1.3 | 0.2 | | | 2.0 | 0.5 | 0 | | |
| | 50 | 2.9 | 0.6 | | | | 1.7 | 0.2 | | | |
| | 100 | 3.1 | | | | | 1.6 | | | | |

TABLE III

| Compound No. | | Lesion scores: *Eimeria acervulina** monensin | | | | | | Lesion scores: *Eimeria tenella** monensin | | | | | |
|---|---|---|---|---|---|---|---|---|---|---|---|---|---|
| C.1. | | 0 | 20 | 40 | 80 | 120 | 160 | 0 | 20 | 40 | 80 | 120 | 160 |
| | 0 | 2.9 | 2.8 | 1.7 | 0.2 | 0 | 0 | 2.9 | 2.7 | 1.3 | 0.3 | 0.1 | 0 |
| | 40 | 3.1 | | 1.0 | 0.1 | | | 2.4 | | 1.3 | 0.6 | | |
| | 80 | 2.7 | | 1.1 | 0.1 | | | 2.0 | | 1.5 | 0.1 | | |
| | 120 | 2.9 | | | | | | 2.9 | | | | | |
| | 240 | 2.8 | | | | | | 2.6 | | | | | |
| C.5. | | 0 | 20 | 40 | 80 | | | 0 | 20 | 40 | 80 | 120 | 160 |
| | 0 | 2.9 | 2.8 | 1.7 | 0.2 | | | 2.9 | 2.7 | 1.3 | 0.3 | 0.1 | 0 |
| | 20 | 2.8 | 2.8 | 1.0 | | | | 3.0 | 2.0 | 0.8 | | | |
| | 40 | 2.6 | 2.2 | 1.1 | 0.3 | | | 3.1 | 1.9 | 1.4 | 0.2 | | |
| | 80 | 2.9 | | | | | | 2.6 | | | | | |
| C.58. | | 0 | 20 | 40 | 80 | 120 | 160 | 0 | 20 | 40 | 80 | 120 | 160 |
| | 0 | 2.9 | 2.8 | 1.7 | 0.2 | 0 | 0 | 2.9 | 2.7 | 1.3 | 0.3 | 0.1 | 0 |
| | 40 | 2.8 | | 0.2 | | | | 2.8 | | 0 | | | |
| C.76. | | 0 | 20 | 40 | 80 | 120 | 160 | 0 | 20 | 40 | 80 | 120 | 160 |
| | 0 | 2.9 | 2.8 | 1.7 | 0.2 | 0 | 0 | 2.9 | 2.7 | 1.3 | 0.3 | 0.1 | 0 |
| | 40 | | | 1.5 | 0.2 | | | | | 1.9 | 0.2 | | |
| | 80 | | | 0.8 | 0.3 | | | | | 1.0 | 0.2 | | |
| | 160 | 3.0 | | | | | | 2.7 | | | | | |

* Infected with 1,000,000 oocysts of *Eimeria acervulina* and 250,000 oocysts of *Eimeria tenella*.

TABLE IV
Lesion scores

| Compound number | | Eimeria acervulina* Eimeria maxima* (intestinal) monensin | | | | Eimeria tenella* (Cecal) monensin | | | |
|---|---|---|---|---|---|---|---|---|---|
| | | 0 | 25 | 50 | 100 | 0 | 25 | 50 | 100 |
| A.1. | 0 | 4.9 | 2.8 | .3 | 0 | 3.3 | 3.3 | .9 | 0 |
| | 25 | 3.7 | .3 | .07 | | 3.2 | .67 | .13 | |
| | 50 | 2.1 | 0 | 0 | | 2.7 | 0 | .07 | |
| | 100 | 1.3 | | | | .6 | | | |
| | | 0 | 25 | 50 | 100 | 0 | 25 | 50 | 100 |
| A.2. | 0 | 5.93 | 3.7 | 2.1 | 0 | 2.83 | 3.1 | 1.2 | .47 |
| | 25 | 1.72 | .33 | .13 | | 1.78 | .4 | 0 | |
| | 50 | .45 | 0 | 0 | | .55 | .2 | 0 | |
| | 100 | 0 | | | | 0 | | | |

* Infected with 500,000 oocysts of *Eimeria acervulina*, 60,000 oocysts of *Eimeria maxima*, and 40,000 oocysts of *Eimeria tenella*. Test conducted with three replicates.

Demonstration of the synergistic effect possessed by the preferred carbanilides of the invention, i.e. nicarbazin or 4,4'-dinitrocarbanilide alone, in combination with the polyether was obtained as follows: one-week-old broiler chicks were allotted to five-bird cages and were fed a medicated or control ration, typically for one day, prior to infection with oocysts of a coccidiosis-causing organism. The chicks were maintained on their respective rations for a period of time, typically seven days. Generally, there were from three to six replicates per treatment. Anticoccidial efficacy was typically determined by the lesion scores, but other measures of efficacy were employed in many of the tests. In determining lesion scores, the birds were sacrificed and the severity of lesions scored on a 0—4 scale, with lesion-free birds scored as 0, extremely severe infections scored as 4, and intermediate degrees of infection scored as 1, 2, or 3. The scores of all birds which received a given treatment were averaged.

In those evaluations where data is reported with superscript letters, data not followed by a common letter are significantly different ($P \leq .05$).

The results of evaluations follows.

Test 1: *Eimeria acervulina* (strain FS-254), inoculated with 200,000 oocysts.

Lesion scores

| | ppm | 0 | monensin 100 |
|---|---|---|---|
| | 0 | $3.16^{c}$ | $1.58^{b}$ |
| nicarbazin | 125 | $1.60^{b}$ | $0^{a}$ |

Test 2: *Eimeria tenella* (FS-226), inoculated with 100,000 oocysts.

Lesion scores

| | ppm | 0 | monensin 100 |
|---|---|---|---|
| | 0 | $3.16^{c}$ | $1.09^{b}$ |
| nicarbazin | 125 | $1.24^{b}$ | $0.18^{a}$ |

Test 3: *Eimeria acervulina* (strains FS-254), inoculated with 40,000 oocysts

Lesion scores

| | ppm | monensin 0 | 60 | 100 |
|---|---|---|---|---|
| | 0 | $3.36^{e}$ | $0.95^{c}$ | $0.80^{c}$ |
| nicarbazin | 60 | $1.05^{cd}$ | $0^{a}$ | $0^{a}$ |
| | 100 | $0.50^{b}$ | | |

Test 4: *Eimeria acervulina* (strain FS-254), inoculated with 200,000 oocysts.

Lesion scores

| | ppm | monensin 0 | 100 |
|---|---|---|---|
| | 0 | $3.45^{c}$ | $1.70^{b}$ |
| nicarbazin | 75 | $2.95^{c}$ | $0^{a}$ |

Test 5: *Eimeria tenella* (strain FS-257), inoculated with 100,000 oocysts

Lesion scores

| | ppm | monensin 0 | 100 |
|---|---|---|---|
| | 0 | $3.46^{c}$ | $1.40^{b}$ |
| nicarbazin | 125 | $1.20^{b}$ | $0.15^{a}$ |

Test 6: *Eimeria acervulina* (strain FS-254), inoculated with 1,000,000 oocysts

Lesion scores

| | ppm | monensin 0 | 60 | 100 |
|---|---|---|---|---|
| | 0 | $3.45^{d}$ | $1.85^{c}$ | $1.15^{b}$ |
| nicarbazin | 60 | $2.95^{d}$ | $0.50^{a}$ | $0.11^{a}$ |
| | 100 | $3.00^{d}$ | | |

Test 7: Combination of *Eimeria acervulina* and *Eimeria maxima* (culture FS-266), 500,000 oocysts.

Lesion scores*

| | ppm | monensin 0 | 40 | 80 | 120 |
|---|---|---|---|---|---|
| | 0 | $5.2^{c}$ | $1.8^{b}$ | $0.5^{a}$ | $0.1^{a}$ |
| nicarbazin | 40 | $1.8^{b}$ | $0.4^{a}$ | $0.4^{a}$ | |
| | 80 | $1.1^{ab}$ | $0.3^{a}$ | | |

* Lesion scores were determined at three locations, anterior, mid-, and posterior portions of the small intestine, scored on 0—4 in each section and expressed as the total.

Test 8: Combination of *Eimeria acervulina* (strain FS-254), 250,000 oocysts, and *Eimeria tenella* (strain FS-257), 50,000 oocysts. One group of birds was sacrificed at 5 days; the other group was used to evaluate oocyst production and then sacrificed at 7 days.

Intestinal lesion scores at 5-days (*Eimeria acervulina*)

| | | monensin | | | | | |
|---|---|---|---|---|---|---|---|
| | ppm | 0 | 20 | 40 | 60 | 80 | 100 |
| | 0 | $3.70^{f}$ | | | $1.50^{cde}$ | $1.13^{bcd}$ | $1.50^{cde}$ |
| nicarbazin | 20 | | $1.63^{de}$ | $1.20^{bcd}$ | $1.40^{cde}$ | $0.40^{a}$ | |
| | 40 | | $1.00^{bc}$ | $0.70^{ab}$ | $0.60^{ab}$ | | |
| | 60 | $1.83^{e}$ | $1.10^{bcd}$ | $0.70^{ab}$ | $0.40^{a}$ | | |
| | 80 | $1.60^{cde}$ | $0.80^{ab}$ | | | | |
| | 100 | $1.50^{cde}$ | | | | | |

Cecal lesion scores at 7-days (*Eimeria tenella*)

| | | monensin | | | | | |
|---|---|---|---|---|---|---|---|
| | ppm | 0 | 20 | 40 | 60 | 80 | 100 |
| | 0 | $3.05^{f}$ | | | $2.90^{f}$ | $2.00^{e}$ | $1.21^{cd}$ |
| nicarbazin | 20 | | $2.86^{f}$ | $1.90^{de}$ | $1.20^{cd}$ | $0.55^{abc}$ | |
| | 40 | | $1.15^{c}$ | $0.92^{bc}$ | $0.75^{abc}$ | $0^{a}$ | |
| | 60 | $2.05^{e}$ | $1.00^{bc}$ | $0.65^{bc}$ | $0.30^{ab}$ | | |
| | 80 | $0.75^{abc}$ | $0.65^{abc}$ | $0.10^{a}$ | | | |
| | 100 | $0.66^{abc}$ | | | | | |

Average oocyst passage/Bird ($\times 10^{6}$)*

| | | monensin | | | | | |
|---|---|---|---|---|---|---|---|
| | ppm | 0 | 20 | 40 | 60 | 80 | 100 |
| | 0 | 64.8 | | | 40.9 | 53.3 | 1.1 |
| nicarbazin | 20 | | 64.6 | 55.6 | 7.5 | 2.0 | |
| | 40 | | 17.1 | 22.9 | 8.7 | 0.7 | |
| | 60 | 66.8 | 7.3 | 4.4 | 0.9 | | |
| | 80 | 42.9 | 1.4 | 4.7 | | | |
| | 100 | 18.7 | | | | | |

* for a 48-hour period, beginning on the 5th day following inoculation and continuing through to sacrifice.

Test 9: *Eimeria acervulina* (strain FS-273) inoculated with 1,150,000 oocysts.

Lesion scores

| | | narasin | | | |
|---|---|---|---|---|---|
| | ppm | 0 | 25 | 50 | 100 |
| | 0 | $3.6^{f}$ | $3.3^{f}$ | $2.4^{e}$ | $1.6^{d}$ |
| nicarbazin | 25 | $3.6^{f}$ | $3.2^{f}$ | $0.6^{b}$ | |
| | 50 | $3.6^{f}$ | $1.1^{c}$ | $0.1^{a}$ | |
| | 100 | $1.8^{d}$ | | | |

Average survivor weight gain in grams

| | | narasin | | | |
|---|---|---|---|---|---|
| | ppm | 0 | 25 | 50 | 100 |
| | 0 | 126.7$^{a}$ | 159.6$^{c}$ | 193.3$^{de}$ | 183.4$^{d}$ |
| nicarbazin | 25 | 142.7$^{b}$ | 188.3$^{d}$ | 212.9$^{fg}$ | |
| | 50 | 159.1$^{c}$ | 203.7$^{ef}$ | 216.8$^{fg}$ | |
| | 100 | 188.3$^{d}$ | | | |

(noninfected, nonmedicated controls=219 grams)

Average feed/gain

| | | narasin | | | |
|---|---|---|---|---|---|
| | ppm | 0 | 25 | 50 | 100 |
| | 0 | 2.23$^{d}$ | 1.96$^{bc}$ | 1.62$^{a}$ | 1.60$^{a}$ |
| nicarbazin | 25 | 2.04$^{c}$ | 1.64$^{a}$ | 1.49$^{a}$ | |
| | 50 | 1.89$^{b}$ | 1.50$^{a}$ | 1.50$^{a}$ | |
| | 100 | 1.61$^{a}$ | | | |

(noninfected nonmedicated controls=1.49$^{a}$)

Comprehensive anticoccidial indices*

| | | narasin | | | |
|---|---|---|---|---|---|
| | ppm | 0 | 25 | 50 | 100 |
| | 0 | 1.38(0) | 1.68(22) | 2.09(52) | 2.17(58) |
| nicarbazin | 25 | 1.49(8) | 1.91(39) | 2.52(84) | |
| | 50 | 1.62(17) | 2.39(75) | 2.66(94) | |
| | 100 | 2.16(57) | | | |

(noninfected, nonmedicated controls=2.74 (100))

*Index=[growth and survival ratio]–[average lesion score/X].

Where: X=4/[.25×ave. growth and survival ratio of noninfected nonmedicated controls]. Growth and survival ratio=[pen weight at termination/pen weight at initiation], adjusted for mortality due to causes other than coccidiosis. Average of five replicates per treatment. The number in parentheses is the percent of optimum anticoccidial activity=[index of infected medicated group–index of infected controls]/[index of noninfected nonmedicated group–index of infected controls]×100.

Test 10: *Eimeria acervulina* (strain FS-254), inoculated with 250,000 oocysts.

Lesion scores

| | | monensin | | |
|---|---|---|---|---|
| | ppm | 0 | 50 | 100 |
| | 0 | 2.38$^{e}$ | 1.36$^{cd}$ | 0.73$^{abc}$ |
| nicarbazin | 60 | 1.80$^{de}$ | 0.44$^{ab}$ | 0.17$^{a}$ |
| | 120 | 1.00$^{bc}$ | | |

Average oocyst passage/bird ($\times 10^6$)*

| | ppm | monensin 0 | 50 | 100 |
|---|---|---|---|---|
| | 0 | 37.4 | 14.8 | 16.0 |
| nicarbazin | 60 | 44.5 | 0.7 | 2.1 |
| | 120 | 44.5 | | |

* for a 24-hour period, beginning on the 5th day following inoculation and continuing through to sacrifice on the 6th day.

Test 11: *Eimeria tenella* (strain FS-286), inoculated with 125,000 oocysts.

Percent mortality attributable to coccidiosis

| | | monensin 0 | 25 | 50 | 100 |
|---|---|---|---|---|---|
| | 0 | $32^{d}$ | $24^{cd}$ | $8^{ab}$ | $0^{a}$ |
| nicarbazin | 25 | $16^{bc}$ | $0^{a}$ | $0^{a}$ | |
| | 50 | $4^{ab}$ | $0^{a}$ | $0^{a}$ | |
| | 100 | $0^{a}$ | | | |

noninfected nonmedicated controls=$0^{a}$

Average survivor weight gain in grams

| | | monensin 0 | 25 | 50 | 100 |
|---|---|---|---|---|---|
| | 0 | $153.4^{a}$ | $165.8^{ab}$ | $198.7^{cd}$ | $220.3^{de}$ |
| nicarbazin | 25 | $185.6^{bc}$ | $242.5^{ef}$ | $227.9^{e}$ | |
| | 50 | $224.2^{e}$ | $246.4^{ef}$ | $244.4^{ef}$ | |
| | 100 | $237.8^{ef}$ | | | |

noninfected nonmedicated controls=$258.2^{f}$

Average feed/gain

| | | monensin 0 | 25 | 50 | 100 |
|---|---|---|---|---|---|
| | 0 | * | $1.64^{cd}$ | $1.68^{d}$ | $1.53^{bc}$ |
| nicarbazin | 25 | $1.79^{d}$ | $1.41^{a}$ | $1.44^{a}$ | |
| | 50 | $1.50^{abc}$ | $1.43^{a}$ | $1.42^{a}$ | |
| | 100 | $1.44^{ab}$ | | | |

noninfected nonmedicated controls=$1.48^{ab}$

* no data because of mortality which occurred in all replicates

Average cecal lesion score per bird

| | | monensin | | | |
|---|---|---|---|---|---|
| | | 0 | 25 | 50 | 100 |
| | 0 | 3.9[e] | 3.8[de] | 3.3[d] | 0.8[ab] |
| nicarbazin | 25 | 3.8[de] | 2.1[c] | 0.8[ab] | |
| | 50 | 3.3[d] | 0.4[a] | 0.3[a] | |
| | 100 | 1.3[b] | | | |

Comprehensive anticoccidial indices*

| | | monensin | | | |
|---|---|---|---|---|---|
| | | 0 | 25 | 50 | 100 |
| | 0 | 0.80(0) | 1.08(13) | 1.75(43) | 2.56(80) |
| nicarbazin | 25 | 1.34(24) | 2.54(79) | 2.69(85) | |
| | 50 | 2.07(58) | 2.88(94) | 2.93(96) | |
| | 100 | 2.70(86) | | | |

noninfected nonmedicated controls=3.01 (100)

* for method of calculation, see Test 9, above.

Test 12: *Eimeria tenella* (strain FS-226-A-204R, a strain propagated in the presence of 15 ppm A-204 for 13 generations prior to use in this experiment), inoculated with 130,000 oocysts.

Mortality attributable to coccidiosis

| | | narasin | | |
|---|---|---|---|---|
| | | 0 | 25 | 50 |
| | 0 | 13.3[b] | 6.7[a] | 6.7[a] |
| nicarbazin | 25 | 0[a] | 0[a] | 0[a] |
| | 50 | 0[a] | 0[a] | 0[a] |

noninfected nonmedicated controls=0[a]

Average survivor weight gain in grams

| | | narasin | | |
|---|---|---|---|---|
| | | 0 | 25 | 50 |
| | 0 | 172.2[a] | 243.4[c] | 239.1[c] |
| nicarbazin | 25 | 198.4[b] | 238.1[c] | 234.1[c] |
| | 50 | 231.7[c] | 238.7[c] | 244.9[c] |

noninfected nonmedicated controls=239.7[c]

Average feed/gain

| | | narasin | | |
|---|---|---|---|---|
| | | 0 | 25 | 50 |
| | 0 | 1.88[b] | 1.51[a] | 1.48[a] |
| nicarbazin | 25 | 1.75[b] | 1.57[a] | 1.51[a] |
| | 50 | 1.57[a] | 1.56[a] | 1.49[a] |

noninfected nonmedicated controls=1.58[a]

Average cecal lesion score per bird

| | | narasin 0 | 25 | 50 |
|---|---|---|---|---|
| | 0 | 3.8[d] | 2.7[c] | 1.5[b] |
| nicarbazin | 25 | 3.5[d] | 0.6[a] | 0.2[a] |
| | 50 | 2.5[c] | 0.3[a] | 0[a] |

Comprehensive anticoccidial indices*

| | | narasin 0 | 25 | 50 |
|---|---|---|---|---|
| | 0 | 1.36(0) | 2.24(55) | 2.40(65) |
| nicarbazin | 25 | 1.92(35) | 2.85(93) | 2.80(90) |
| | 50 | 2.43(67) | 2.86(94) | 2.92(98) |

noninfected nonmedicated controls=2.96 (100)

* for method of calculation, see Test 9, above.

Test 13: *Eimeria acervulina* (strain FS-254), inoculated with 1,000,000 oocysts.

Average survivor weight gain in grams

| | | narasin 0 | 25 | 50 |
|---|---|---|---|---|
| | 0 | 147.3[a] | 186.7[c] | 199.0[cd] |
| nicarbazin | 25 | 157.3[ab] | 196.9[cd] | 205.5[de] |
| | 50 | 162.6[b] | 215.9[e] | 200.7[d] |

noninfected nonmedicated controls=217.1[e]

Average feed/gain

| | | narasin 0 | 25 | 50 |
|---|---|---|---|---|
| | 0 | 2.09[c] | 1.82[b] | 1.62[a] |
| nicarbazin | 25 | 1.81[b] | 1.57[a] | 1.51[a] |
| | 50 | 1.96[bc] | 1.54[a] | 1.53[a] |

noninfected nonmedicated controls=1.59[a]

Average intestinal lesion score per bird

| | | narasin 0 | 25 | 50 |
|---|---|---|---|---|
| | 0 | 3.4[d] | 2.6[c] | 2.1[c] |
| nicarbazin | 25 | 3.6[d] | 1.4[b] | 0.3[a] |
| | 50 | 3.3[d] | 0.6[a] | 0.1[a] |

Comprehensive anticoccidial indices*

| | | narasin | | |
|---|---|---|---|---|
| | | 0 | 25 | 50 |
| | 0 | 1.58(0) | 2.02(38) | 2.23(57) |
| nicarbazin | 25 | 1.61(3) | 2.36(68) | 2.62(91) |
| | 50 | 1.76(16) | 2.59(88) | 2.60(89) |

noninfected nonmedicated controls=2.73 (100)

* for method of calculation, see Test 9 above.

Test 14: *Eimeria acervulina* (strain FS-273), inoculated with 780,000 oocysts.

Average survivor weight gain in grams

| | | salinomycin | | |
|---|---|---|---|---|
| | | 0 | 25 | 50 |
| | 0 | 155.3[a] | 170.2[ab] | 199.7[cd] |
| nicarbazin | 25 | 164.5[ab] | 210.1[de] | 204.3[de] |
| | 50 | 180.0[bc] | 209.7[de] | 209.6[de] |

noninfected nonmedicated controls=226.8[e]

Average feed/gain

| | | salinomycin | | |
|---|---|---|---|---|
| | | 0 | 25 | 50 |
| | 0 | 1.80[d] | 1.72[bcd] | 1.63[abcd] |
| nicarbazin | 25 | 1.78[cd] | 1.56[abcd] | 1.50[ab] |
| | 50 | 1.68[abcd] | 1.53[abc] | 1.43[a] |

noninfected nonmedicated controls=1.42[a]

Average intestinal lesion score per bird

| | | salinomycin | | |
|---|---|---|---|---|
| | | 0 | 25 | 50 |
| | 0 | 3.9[d] | 3.7[d] | 2.9[bc] |
| nicarbazin | 25 | 3.9[d] | 2.1[b] | 0.2[a] |
| | 50 | 3.7[cd] | 0.1[a] | 0[a] |

Comprehensive anticoccidial indices*

| | | salinomycin | | |
|---|---|---|---|---|
| | | 0 | 25 | 50 |
| | 0 | 1.54(0) | 1.70(15) | 2.04(45) |
| nicarbazin | 25 | 1.60(6) | 2.23(61) | 2.54(89) |
| | 50 | 1.77(21) | 2.57(91) | 2.60(94) |

noninfected nonmedicated controls=2.67 (100)

* for method of calculation, see Test 9, above.

Test 15: *Eimeria acervulina* (strain FS-273), inoculated with 780,000 oocysts.

Average survivor weight gain in grams

| | | Ionomycin 0 | 25 | 50 |
|---|---|---|---|---|
| | 0 | $145.7^{a}$ | $162.4^{abc}$ | $179.5^{cde}$ |
| nicarbazin | 25 | $154.0^{ab}$ | $184.3^{de}$ | $198.6^{ef}$ |
| | 50 | $171.8^{bcd}$ | $195.9^{ef}$ | $212.7^{f}$ |

noninfected nonmedicated controls=$210.9^{f}$

Average feed/gain

| | | Ionomycin 0 | 25 | 50 |
|---|---|---|---|---|
| | 0 | $1.89^{d}$ | $1.68^{bc}$ | $1.57^{ab}$ |
| nicarbazin | 25 | $1.80^{cd}$ | $1.55^{ab}$ | $1.49^{a}$ |
| | 50 | $1.68^{bc}$ | $1.46^{a}$ | $1.46^{a}$ |

noninfected nonmedicated controls=$1.43^{a}$

Average intestinal lesion score per bird

| | | Ionomycin 0 | 25 | 50 |
|---|---|---|---|---|
| | 0 | $3.3^{d}$ | $3.1^{d}$ | $1.9^{c}$ |
| nicarbazin | 25 | $2.8^{d}$ | $2.1^{c}$ | $0.7^{b}$ |
| | 50 | $2.3^{c}$ | $0.5^{b}$ | $0^{a}$ |

noninfected nonmedicated controls=0

Comprehensive anticoccidial indices*

| | | Ionomycin 0 | 25 | 50 |
|---|---|---|---|---|
| | 0 | 1.61(0) | 1.77(16) | 2.12(48) |
| nicarbazin | 25 | 1.76(15) | 2.14(50) | 2.48(83) |
| | 50 | 1.97(35) | 2.49(83) | 2.62(96) |

noninfected nonmedicated controls=2.66(100)

*for method of calculation, see Test 9, above.

Test 16: *Eimeria tenella* (strain FS-226-A204R), inoculated with 130,00 oocysts.

Mortality attributable to coccidiosis

| | | A-204 0 | 5 | 10 |
|---|---|---|---|---|
| | 0 | $13.3^{b}$ | $6.7^{a}$ | $0^{a}$ |
| nicarbazin | 50 | $0^{a}$ | $0^{a}$ | $0^{a}$ |
| | 100 | $0^{a}$ | $0^{a}$ | $0^{a}$ |

noninfected nonmedicated controls=$0^{a}$

Average survivor weight gain in grams

| | | A-204 | | |
|---|---|---|---|---|
| | | 0 | 5 | 10 |
| | 0 | $200.9^{ab}$ | $190.2^{a}$ | $225.7^{bc}$ |
| nicarbazin | 50 | $235.5^{c}$ | $243.4^{c}$ | $241.6^{c}$ |
| | 100 | $232.8^{c}$ | $227.2^{bc}$ | $236.2^{c}$ |

noninfected nonmedicated controls=$256.3^{c}$

Average feed/gain

| | | A-204 | | |
|---|---|---|---|---|
| | | 0 | 5 | 10 |
| | 0 | $1.75^{c}$ | $1.74^{bc}$ | $1.63^{abc}$ |
| nicarbazin | 50 | $1.50^{a}$ | $1.49^{a}$ | $1.48^{a}$ |
| | 100 | $1.54^{ab}$ | $1.67^{abc}$ | $1.51^{a}$ |

noninfected nonmedicated controls=$1.48^{a}$

Average cecal lesion score per bird

| | | A-204 | | |
|---|---|---|---|---|
| | | 0 | 5 | 10 |
| | 0 | $3.8^{e}$ | $3.1^{e}$ | $2.3^{d}$ |
| nicarbazin | 50 | $1.9^{d}$ | $0.5^{bc}$ | $0^{a}$ |
| | 100 | $0.8^{c}$ | $0.1^{ab}$ | $0^{a}$ |

Comprehensive anticoccidial indices*

| | | A-204 | | |
|---|---|---|---|---|
| | | 0 | 5 | 10 |
| | 0 | 1.42(0) | 1.71(20) | 2.25(57) |
| nicarbazin | 50 | 2.42(70) | 2.70(90) | 2.79(96) |
| | 100 | 2.53(78) | 2.65(86) | 2.72(91) |

noninfected nonmedicated controls=2.85 (100)

* for method of calculation, see Test 9, above.

The following test was conducted in two-week-old straight run turkeys.

Test 17: *Eimeria meleagrimitis* (strain FS-230-MR, pass #7)

Mortality attributable to coccidiosis*

| | | monensin | | | | | |
|---|---|---|---|---|---|---|---|
| | | 0 | 40 | 60 | 80 | 100 | 120 |
| | 0 | 6.2 | | 6.2 | 0 | 0 | 0 |
| nicarbazin | 40 | | 0 | 0 | | | |
| | 60 | 6.2 | 0 | 0 | | | |
| | 80 | 6.2 | | | | | |
| | 100 | 0 | | | | | |
| | 120 | 0 | | | | | |

noninfected nonmedicated controls=0

*There were no significant differences among treatments P<.05.

Average survivor weight gain in grams

| | | monensin | | | | | |
|---|---|---|---|---|---|---|---|
| | | 0 | 40 | 60 | 80 | 100 | 120 |
| | 0 | $100.7^{ab}$ | | $118.8^{bcd}$ | $124.8^{cd}$ | $134.1^{de}$ | $140.3^{de}$ |
| nicarbazin | 40 | | $135.8^{de}$ | $164.6^{f}$ | | | |
| | 60 | $98.8^{ab}$ | $150.4^{ef}$ | $169.3^{f}$ | | | |
| | 80 | $95.7^{a}$ | | | | | |
| | 100 | $99.2^{ab}$ | | | | | |
| | 120 | $112.4^{abc}$ | | | | | |

noninfected nonmedicated controls=$221.6^{g}$

Average feed/gain

| | | monensin | | | | | |
|---|---|---|---|---|---|---|---|
| | | 0 | 40 | 60 | 80 | 100 | 120 |
| | 0 | $2.42^{fgh}$ | | $2.17^{def}$ | $2.16^{def}$ | $2.07^{de}$ | $2.00^{bcd}$ |
| nicarbazin | 40 | | $2.04^{cde}$ | $1.80^{bc}$ | | | |
| | 60 | $2.63^{h}$ | $1.90^{bcd}$ | $1.77^{b}$ | | | |
| | 80 | $2.55^{gh}$ | | | | | |
| | 100 | $2.53^{gh}$ | | | | | |
| | 120 | $2.29^{efg}$ | | | | | |

noninfected nonmedicated controls=$1.77^{a}$

Growth and survival ratios*

| | | monensin | | | | | |
|---|---|---|---|---|---|---|---|
| | | 0 | 40 | 60 | 80 | 100 | 120 |
| | 0 | 1.35(0) | | 1.42(11) | 1.53(30) | 1.59(39) | 1.61(44) |
| nicarbazin | 40 | | 1.59(40) | 1.71(59) | | | |
| | 60 | 1.34(0) | 1.64(48) | 1.71(60) | | | |
| | 80 | 1.33(0) | | | | | |
| | 100 | 1.42(12) | | | | | |
| | 120 | 1.48(22) | | | | | |

noninfected nonmedicated controls=1.95 (100)

* Growth and survival ratio (GSR)=pen weight at termination/pen weight at initiation, adjusted for mortality due to causes other than coccidiosis.
Data expressed as the average of four replicates/treatment.
Numbers in parenthesis are the % of optimum anticoccidial activity=[GSR of infected medicated group–GSR of infected controls][GSR of noninfected nonmedicated group–GSR of infected controls]×100.

The following additional tests were conducted in chickens, as described above.

Test 18: *Eimeria acervulina* (strain FS-254), inoculated with 1,000,000 oocysts.

Lesion scores

| | | lasalocid | | | |
|---|---|---|---|---|---|
| | ppm | 0 | 25 | 50 | 100 |
| | 0 | $2.7^{ef}$ | $2.4^{cdef}$ | $2.7^{def}$ | $1.6^{bc}$ |
| nicarbazin | 25 | $2.8^{f}$ | $2.3^{bcdef}$ | $1.0^{ab}$ | |
| | 50 | $2.0^{bcde}$ | $1.7^{bcd}$ | $0.1^{a}$ | |
| | 100 | $2.0^{bcde}$ | | | |

Average oocyst passage/bird ($\times 10^6$)*

| | | lasalocid | | | |
|---|---|---|---|---|---|
| | ppm | 0 | 25 | 50 | 100 |
| | 0 | $51^{ab}$ | $92^{ab}$ | $93^{ab}$ | $112^{ab}$ |
| nicarbazin | 25 | $152^{b}$ | $10^{ab}$ | $12^{ab}$ | |
| | 50 | $83^{ab}$ | $15^{ab}$ | $5^{a}$ | |
| | 100 | $44^{ab}$ | | | |

* for a 24-hour period, 120—144 hours post inoculation.

Test 19: *Eimeria tenella* (strain FS-257), inoculated with 200,000 oocysts.

Lesion scores

| | | lasalocid | | | |
|---|---|---|---|---|---|
| | ppm | 0 | 25 | 50 | 100 |
| nicarbazin | 0 | $4.0^{c}$ | $4.0^{c}$ | $3.9^{c}$ | $3.6^{c}$ |
| | 25 | $3.9^{c}$ | $3.4^{c}$ | $2.5^{b}$ | |
| | 50 | $3.6^{c}$ | $1.9^{ab}$ | $1.6^{a}$ | |
| | 100 | $2.0^{ab}$ | | | |

Average oocyst passage/bird ($\times 10^{6}$)*

| | | lasalocid | | | |
|---|---|---|---|---|---|
| | ppm | 0 | 25 | 50 | 100 |
| nicarbazin | 0 | $25^{ab}$ | $51^{c}$ | $49^{c}$ | $20^{ab}$ |
| | 25 | $31^{b}$ | $13^{ab}$ | $<1^{a}$ | |
| | 50 | $5^{a}$ | $0^{a}$ | $0^{a}$ | |
| | 100 | $0^{a}$ | | | |

* for a 24-hour period, 144—168 hours postinoculation

Test 20: Combination of *Eimeria acervulina* (strain FS-254), 300,000 oocysts, and *Eimeria tenella* (strain FS-287), 88,000 oocysts.

Lesion scores

Intestinal (*Eimeria acervulina*) /(*Eimeria tenella*) Cecal

| | | monensin | | | |
|---|---|---|---|---|---|
| | ppm | 0 | 25 | 50 | 100 |
| 4,4'-dinitro-carbanilide | 0 | $2.1^{fg}/3.9^{g}$ | $1.9^{efg}/3.9^{g}$ | $1.7^{efg}/2.7^{efg}$ | $0.7^{abcd}/1.7^{bcde}$ |
| | 50 | | | $0^{a}/0.1^{a}$ | |
| | 100 | $0.3^{ab}/1.1^{abcd}$ | | | |

Test 21: *Eimeria tenella* (strain FS-283), 125,000 oocysts.

Lesion scores

| | | monensin | | |
|---|---|---|---|---|
| | ppm | 25 | 50 | 100 |
| 4,4'-dinitro-carbanilide | 0 | $2.8^{fg}$ | $2.1^{cdefg}$ | $0.7^{ab}$ |
| | 50 | | $1.1^{abcd}$ | |
| | 100 | $2.4^{defg}$ | | |

Test 22: Combination of *Eimeria acervulina* (strain FS-280), 430,000 oocysts, and *Eimeria tenella* (strain FS-260), 43,000 oocysts.

| | | Lesion scores Intestinal (*Eimeria acervulina*)/(Eimeria tenella) Cecal monensin | | | | | |
|---|---|---|---|---|---|---|---|
| | ppm | 0 | 25 | 50 | 75 | 100 | 125 |
| | 0 | $2.6^{d}/4.0^{f}$ | $2.3^{d}/3.9^{f}$ | $1.5^{c}/2.5^{e}$ | $0.7^{b}/1.2^{cd}$ | $0.2^{a}/0.4^{ab}$ | $0.3^{a}/0.7^{abc}$ |
| 4,4′-dinitro-carbanilide | 25 | $2.6^{d}/3.9^{f}$ | $0.8^{b}/2.2^{3}$ | $0.2^{a}/0.3^{a}$ | $0^{a}/0^{a}$ | $0^{a}/0^{a}$ | |
| | 50 | $0.9^{b}/3.6^{f}$ | $0^{a}/0.2^{a}$ | $0^{a}/0^{a}$ | $0^{a}/0^{a}$ | | |
| | 75 | $0.2^{a}/2.3^{e}$ | $0^{a}/0^{a}$ | $0^{a}/0^{a}$ | | | |
| | 100 | $0^{a}/1.6^{d}$ | $0^{a}/0.1^{a}$ | | | | |
| | 125 | $0^{a}/1.0^{bcd}$ | | | | | |

Test 23: *Eimeria acervulina* (750,000 oocysts) A32887 (K41)

| | Lesion scores | | | | |
|---|---|---|---|---|---|
| | ppm | 0 | 25 | 50 | 100 |
| | 0 | 3.07 | 3.20 | 1.68 | 0.48 |
| Nicarbazin | 25 | 2.43 | 0 | | |
| | 50 | 0 | | | |
| | 100 | 0 | | | |

Test 24: *Eimeria tenella* (75,000 oocysts) A32887 (K41)

| | Lesion scores | | | | |
|---|---|---|---|---|---|
| | ppm | 0 | 25 | 50 | 100 |
| | 0 | 1.73 | 2.0 | 0.9 | 0.22 |
| Nicarbazin | 25 | 1.87 | 0.2 | | |
| | 50 | 0.6 | | 0 | |
| | 100 | 0.67 | | | |

**Claims**

1. An anticoccidial composition for consumption by an avian species; characterized in that the composition comprises as active ingredients a polyether antibiotic selected from monensin (factors A, B, and C), laidlomycin, grisorixin, lenoremycin, salinomycin, narasin, lonomycin, alborixin, antibiotic A204, A32887 (K41), etheromycin, lasalocid (factors A, B, C, D, and E), isolasalocid A, lysocellin, mutalomycin, or antibiotic A23187 and a carbanilide derivative, the ratio of carbanilide to polyether being in the range of from 40:1 to 1:20, associated with a carrier or feedstuff.

2. An anticoccidial composition as claimed in claim 1, wherein the polyether antibiotic is monensin, narasin, lasalocid, salinomycin or A-204.

3. An anticoccidial composition as claimed in claim 2 wherein the polyether antibiotic is monensin.

4. An anticoccidial composition as claimed in claim 2 wherein the polyether antibiotic is narasin.

5. An anticoccidial composition as claimed in claim 4 wherein the ratio is in the range of from 10:1 to 1:10.

6. An anticoccidial composition as claimed in claim 4 wherein the ratio is in the range of from 2:1 to 1:2.

7. An anticoccidial composition as claimed in any one of claims 1 to 6 wherein the carbanilide possesses the structural formula (I):

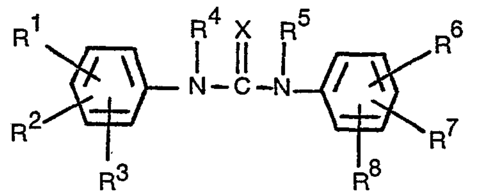

(I)

wherein $R^1$, $R^2$ and $R^3$ are the same or different and can each represent hydrogen; halogen; cyano; amino; nitro; $C_{1-6}$alkyl; $C_{1-4}$alkoxy; $C_{2-4}$alkanoylamino; $C_{1-4}$alkylthio; $C_{1-4}$haloalkyl; $C_{1-4}$haloalkoxy; $C_{1-4}$haloalkylthio; $C_{1-6}$alkoxycarbonyl; $C_{2-4}$haloalkenyloxy; $C_{1-4}$alkoxycarbonylthio; $C_{1-4}$alkylsulfonyl; $C_{1-4}$haloalkylsulfonyl; fluorosulfonyl; phenoxy optionally substituted by halogen, $C_{1-4}$haloalkyl or nitro; phenoxycarbonyl optionally substituted by $C_{1-4}$alkyl; phenyl; or phenyl-sulfonyl optionally substituted by nitro, acetamido, isopropylideneamino or a group of the formula

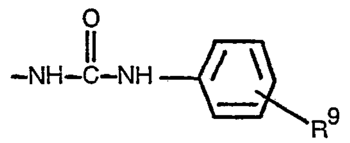

where $R^9$ is $C_{1-4}$haloalkylthio or $C_{2-4}$haloalkenyloxy; $R^4$ and $R^5$ are the same or different and can each represent hydrogen or $C_{1-4}$alkyl; $R^6$, $R^7$ and $R^8$ are the same or different and can each represent hydrogen; halo; cyano; amino; $C_{2-4}$alkanoylamino; N-$C_{1-4}$alkyl $C_{2-4}$alkanoylamino; nitro; $C_{1-6}$alkyl; $C_{1-4}$alkoxy; $C_{1-4}$alkylthio, $C_{1-4}$haloalkyl; $C_{1-4}$haloalkoxy, $C_{1-4}$haloalkylthio, $C_{2-4}$haloalkenyloxy; $C_{1-4}$haloalkylsulfonyl; $C_{1-6}$alkoxycarbonyl, aminosulfonyl or benzoyl; provided that at least one of $R^1$, $R^2$, $R^3$, $R^6$, $R^7$ and $R^8$ are other than hydrogen, and complexes of such compounds with 2-hydroxy-4,6-dimethylpyrimidine.

8. An anticoccidial composition as claimed in any one of claims 1 to 7, wherein the carbanilide derivative is Nicarbazin.

9. A composition according to claim 1 to 8 wherein the carbanilide is present in a concentration of from about 10 to about 250 ppm.

10. A composition according to claim 9, wherein the concentration is from about 25 to about 100 ppm.

11. A composition according to any one of claims 1 to 10 which contains from 15 ppm to 25% by weight of active ingredients.

12. A composition according to any one of claims 1, 2 or 4 to 11 which contains from about 25 to about 100 ppm of narasin.

13. A composition according to any one of claims 1 to 12 which comprises a total feed.

14. An anticoccidial composition for consumption by an avian species characterized in that it contains as active ingredients narasin and nicarbazin, associated with a carrier or feedstuff.

15. A composition as claimed in any one of claims 1 to 14 in the form of a premix.

16. A composition as claimed in any one of claims 1 to 15 for use in a method of controlling coccidiosis in poultry which comprises supplying to said poultry the composition as at least part of their feed.

**Patentansprüche**

1. Mittel gegen Kokzidiose zum Verbrauch durch eine Vogelart, dadurch gekennzeichnet, daß dieses Mittel als Wirkstoffe eine Kombination aus einem Polyetherantibioticum ausgewählt aus Monensin (Faktoren A, B und C), Laidlomycin, Grisorixin, Lenoremycin, Salinomycin, Narasin, Lonomycin, Alborixin, Antibioticum A204, A32887 (K41), Etheromycin, Lasalocid (Faktoren A, B, C, D und E), Isolasalocid A, Lysocellin, Mutalomycin oder Antibioticum A23187, und aus einem Carbanilidderivat, wobei das Gewichtsverhältnis von Carbanilidderivat zu Polyetherantibioticum im Bereich von 40 zu 1 bis 1 zu 20 liegt, in Verbindung mit einem Träger oder Futtermittel enthält.

2. Mittel gegen Kokzidiose nach Anspruch 1, dadurch gekennzeichnet, daß es als Polyetherantibioticum Monensin, Narasin, Lasalocid, Salinomycin oder Antibioticum A-204 enthält.

3. Mittel gegen Kokzidiose nach Anspruch 2, dadurch gekennzeichnet, daß es als Polyetherantibioticum Monensin enthält.

4. Mittel gegen Kokzidiose nach Anspruch 2, dadurch gekennzeichnet, daß es als Polyetherantibioticum Narasin enthält.

5. Mittel gegen Kokzidiose nach Anspruch 4, dadurch gekennzeichnet, daß es das Carbanilidderivat und das Polyetherantibioticum in einem Gewichtsverhältnis im Bereich von 10 zu 1 bis 1 zu 10 enthält.

6. Mittel gegen Kokzidiose nach Anspruch 4, dadurch gekennzeichnet, daß es das Carbanilidderivat und das Polyetherantibioticum in einem Gewichtsverhältnis im Bereich von 2 zu 1 bis 1 zu 2 enthält.

7. Mittel gegen Kokzidiose nach einem der Ansprüche 1 bis 6, dadurch gekennzeichnet, daß es ein Carbanilidderivat der allgemeinen Formel I

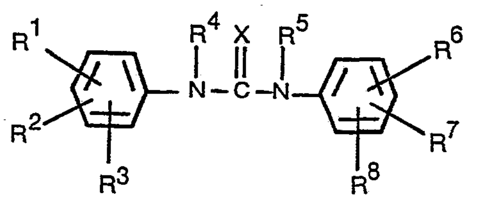

worin $R^1$, $R^2$ und $R^3$ gleich oder verschieden sind und Wasserstoff, Halogen, Cyano, Amino, Nitro, $C_1$—$C_6$-Alkyl, $C_1$—$C_4$-Alkoxy, $C_2$—$C_4$-Alkanoylamino, $C_1$—$C_4$-Alkylthio, $C_1$—$C_4$-Halogenalkyl, $C_1$—$C_4$-Halogenalkoxy, $C_1$—$C_4$-Halogenalkylthio, $C_1$—$C_6$-Alkoxycarbonyl, $C_2$—$C_4$-Halogenalkenyloxy, $C_1$—$C_4$-Alkoxycarbonylthio, $C_1$—$C_4$-Alkylsulfonyl, $C_1$—$C_4$-Halogenalkylsulfonyl, Fluorsulfonyl, Phenoxy, das gegebenenfalls durch Halogen, $C_1$—$C_4$-Halogenalkyl oder Nitro substituiert ist, Phenoxycarbonyl, das gegebenenfalls durch $C_1$—$C_4$-Alkyl substituiert ist, Phenyl oder Phenylsulfonyl, das gegebenenfalls durch Nitro, Acetamido, Isopropylidenamino oder eine Gruppe der allgemeinen Formel

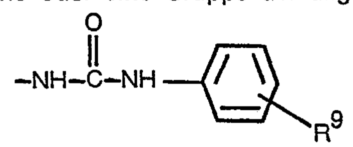

substituiert ist, worin $R^9$ für $C_1$—$C_4$-Halogenalkylthio oder $C_2$—$C_4$-Halogenalkenyloxy steht, bedeuten, $R^4$ und $R^5$ gleich oder verschieden sind und jeweils Wasserstoff oder $C_1$—$C_4$-Alkyl darstellen, $R^6$, $R^7$ und $R^8$ gleich oder verschieden sind und jeweils Wasserstoff, Halogen, Cyano, Amino, $C_2$—$C_4$-Alkanoylamino, N-$C_1$—$C_4$-Alkyl-$C_2$—$C_4$-alkanoylamino, Nitro, $C_1$—$C_6$-Alkyl, $C_1$—$C_4$-Alkoxy, $C_1$—$C_4$-Alkylthio, $C_1$—$C_4$-Halogenalkyl, $C_1$—$C_4$-Halogenalkoxy, $C_1$—$C_4$-Halogenalkylthio, $C_2$—$C_4$-Aalogenalkenyloxy, $C_1$—$C_4$-Halogenalkylsulfonyl, $C_1$—$C_6$-Alkoxycarbonyl, Aminosulfonyl oder Benzoyl sind, mit der Maßgabe, daß wenigstens einer der Substituenten $R^1$, $R^2$, $R^3$, $R^6$, $R^7$ und $R^8$ eine andere Bedeutung als Wasserstoff hat, oder Komplexe solcher Verbindungen mit 2-Hydroxy-4,6-Dimethylpyrimidin enthält.

8. Mittel gegen Kokzidiose nach einem der Ansprüche 1 bis 7, dadurch gekennzeichnet, daß es als Carbanilidderivat Nicarbacin enthält.

9. Mittel nach Anspruch 1 bis 7, dadurch gekennzeichnet, daß es das Carbanilidderivat in einer Konzentration von etwa 10 bis etwa 250 ppm enthält.

10. Mittel nach Anspruch 9, dadurch gekennzeichnet, daß es das Carbanilidderivat in einer Konzentration von etwa 25 bis etwa 100 ppm enthält.

11. Mittel nach einem der Ansprüche 1 bis 10, dadurch gekennzeichnet, daß es die Wirkstoffe in einer Konzentration von 15 ppm bis 25 Gewichtsprozent enthält.

12. Mittel nach einem der Ansprüche 1, 2 oder 4 bis 11, dadurch gekennzeichnet, daß es als Wirkstoff etwa 25 bis etwa 100 ppm Narasin enthält.

13. Mittel nach einem der Ansprüche 1 bis 12, dadurch gekennzeichnet, daß es ein Gesamtfutter enthält.

14. Mittel gegen Kokzidiose zum Verbrauch durch eine Vogelart, dadurch gekennzeichnet, daß es als Wirkstoffe eine Kombination aus Narasin und Nicarbacin in Verbindung mit einem Träger oder Futtermittel enthält.

15. Mittel nach einem der Ansprüche 1 bis 14, dadurch gekennzeichnet, daß es die Form eines Vorfuttergemisches hat.

16. Verwendung des Mittels nach einem der Ansprüche 1 bis 15 zur Bekämpfung von Kokzidiose bei Geflügel, indem man Geflügel wenigstens als Teil ihres Futters ein solches Mittel verabreicht.

**Revendications**

1. Composition anticoccidienne destinée à la consommation par une espèce avienne, caractérisée en ce qu'elle comprend, comme ingrédients actifs, un antibiotique de polyéther choisi parmi la monensine (facteurs A, B et C), la laidlomycine, la grisorixine, la lénorémycine, la salinomycine, la narasine, la lonomycine, l'alborixine, l'antibiotique A204, le A32887 (K41), l'éthéromycine, le lasalocide (facteurs A, B, C, D et E), l'isolasalocide A, la lysocelline, la mutalomycine ou l'antibiotique A23187, ainsi qu'un dérivé de carbanilide, le rapport entre le carbanilide et le polyéther se situant dans l'intervalle compris entre 40:1 et 1:20, en association avec un support ou une pâtée.

2. Composition anticoccidienne selon la revendication 1, caractérisée en ce que l'antibiotique de polyéther est la monensine, la narasine, le lasalocide, la salinomycine ou le A-204.

3. Composition anticoccidienne selon la revendication 2, caractérisée en ce que l'antibiotique de polyéther est la monensine.

4. Composition anticoccidienne selon la revendication 2, caractérisée en ce que l'antibiotique de polyéther est la narasine.

5. Composition anticoccidienne selon la revendication 4, caractérisée en ce que le rapport se situe dans l'intervalle allant de 10:1 à 1:10.

6. Composition anticoccidienne selon la revendication 4, caractérisée en ce que le rapport se situe dans l'intervalle allant de 2:1 à 1:2.

7. Composition anticoccidienne selon l'une quelconque des revendications 1 à 6, caractérisée en ce que le carbanilide possède la formule structurale (I):

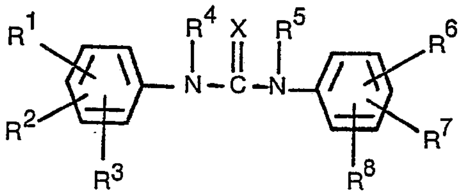

(I)

dans laquelle $R^1$, $R^2$ et $R^3$ sont identiques ou différents et peuvent représenter chacun un atome d'hydrogène; un atome d'halogène; un groupe cyano; un groupe amino; un groupe nitro; un groupe alkyle en $C_1$—$C_6$; un groupe alcoxy en $C_1$—$C_4$; un groupe alcanoyl (en $C_2$—$C_4$)amino; un groupe alkyl (en $C_1$—$C_4$)thio; un groupe haloalkyle en $C_1$—$C_4$; un groupe haloalcoxy en $C_1$—$C_4$; un groupe haloalkyl (en $C_1$—$C_4$)thio; un groupe alcoxy (en $C_1$—$C_6$) carbonyle; un groupe haloalcényl (en $C_2$—$C_4$)oxy; un groupe alcoxy (en $C_1$—$C_4$)carbonylthio; un groupe alkyl (en $C_1$—$C_4$)sulfonyle; un groupe haloalkyl (en $C_1$—$C_4$)sulfonyle; un groupe fluorosulfonyle; un groupe phénoxy éventuellement substitué par un atome d'halogène, un groupe haloalkyle en $C_1$—$C_4$ ou un groupe nitro; un groupe phénoxycarbonyle éventuellement substitué par un groupe alkyle en $C_1$—$C_4$; un groupe phényle; ou un groupe phénylsulfonyle éventuellement substitué par un groupe nitro, un groupe acétamido, un groupe isopropylidèneamino ou un groupe de formule:

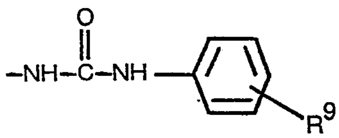

où $R^9$ représente un groupe haloalkyl (en $C_1$—$C_4$)thio ou un groupe halo-alcényl (en $C_2$—$C_4$)oxy; $R^4$ et $R^5$ sont identiques ou différents et peuvent représenter chacun un atome d'hydrogène ou un groupe alkyle en $C_1$—$C_4$; $R^6$, $R^7$ et $R^8$ sont identiques ou différents et peuvent représenter chacun un atome d'hydrogène; un atome d'halogène; un groupe cyano; un groupe amino; un groupe alcanoyl (en $C_2$—$C_4$)-amino; un groupe N-alkyl(en $C_1$—$C_4$)-alcanoyl(en $C_2$—$C_4$)-amino; un groupe nitro; un groupe alkyle en $C_1$—$C_6$; un groupe alcoxy en $C_1$—$C_4$; un groupe alkyl (en $C_1$—$C_4$1-thio, un groupe haloalkyle en $C_1$—$C_4$; un groupe haloalcoxy en $C_1$—$C_4$, un groupe haloalkyl (en $C_1$—$C_4$)-thio, un groupe haloalcényl (en $C_2$—$C_4$)-oxy; un groupe haloalkyl (en $C_1$—$C_4$)-sulfonyle; un groupe alcoxy (en $C_1$—$C_6$)-carbonyle, un groupe aminosulfonyle ou un groupe benzoyle; à condition qu'au moins un des radicaux $R^1$, $R^2$, $R^3$, $R^6$, $R^7$ et $R^8$ soit différent de l'hydrogène, de même que les complexes des composés de ce type avec la 2-hydroxy-4,6-diméthylpyrimidine.

8. Composition anticoccidienne selon l'une quelconque des revendications 1 à 7, caractérisée en ce que le dérivé de carbanilide est la Nicarbazine.

9. Composition selon les revendications 1 à 8, caractérisée en ce que le carbanilide est présent en une concentration comprise entre environ 10 et environ 250 parties par million.

10. Composition selon la revendication 9, caractérisée en ce que la concentration est comprise entre environ 25 et environ 100 parties par million.

11. Composition selon l'une quelconque des revendications 1 à 10, caractérisée en ce qu'elle contient 15 parties par million à 25% en poids d'ingrédients actifs.

12. Composition selon l'une quelconque des revendications 1, 2 ou 4 à 11, caractérisée en ce qu'elle contient environ 25 à environ 100 parties par million de narasine.

13. Composition selon l'une quelconque des revendications 1 à 12, caractérisée en ce qu'elle constitue une nourriture totale.

14. Composition anticoccidienne destinée à la consommation par une espèce avienne, caractérisée en ce qu'elle contient, comme ingrédients actifs, de la narasine et de la nicarbazine, en association avec un support ou une pâtée.

15. Composition selon l'une quelconque des revendications 1 à 14, caractérisée en ce qu'elle est sous la forme d'une prémélange.

16. Composition selon l'une quelconque des revendications 1 à 15, en vue de son utilisation dans un procédé de contrôle de la coccidiose chez la volaille, ce procédé consistant à donner cette composition à la volaille, sous forme d'au moins une partie de sa nourriture.